# EUROPÄISCHE PATENTSCHRIFT

(11) **EP 4 441 136 B1**
(45) Veröffentlichungstag und Bekanntmachung des Hinweises auf die Patenterteilung: **26.11.2025**
(21) Anmeldenummer: 22801992.3
(22) Anmeldetag: 02.11.2022
(51) Int. Cl.: C08K 5/3437, B60C 1/00, C08K 3/04

(54) **VERBINDUNG, KAUTSCHUKMISCHUNG ENTHALTEND DIE VERBINDUNG, FAHRZEUGREIFEN, DER DIE KAUTSCHUKMISCHUNG IN WENIGSTENS EINEM BAUTEIL AUFWEIST, VERFAHREN ZUR HERSTELLUNG DER VERBINDUNG SOWIE VERWENDUNG DER VERBINDUNG ALS ALTERUNGSSCHUTZMITTEL UND/ODER ANTIOXIDATIONSMITTEL UND/ODER FARBSTOFF**
COMPOUND, RUBBER BLEND CONTAINING THE COMPOUND, VEHICLE TIRE COMPRISING THE RUBBER BLEND IN AT LEAST ONE COMPONENT, PROCESS FOR PRODUCING THE COMPOUND, AND USE OF THE COMPOUND AS AN AGEING PROTECTANT AND/OR ANTIOXIDANT AND/OR DYE
COMPOSÉ, MÉLANGE DE CAOUTCHOUC CONTENANT LE COMPOSÉ, PNEU DE VÉHICULE COMPRENANT LE MÉLANGE DE CAOUTCHOUC DANS AU MOINS UN COMPOSANT, PROCÉDÉ DE PRODUCTION DU COMPOSÉ, ET UTILISATION DU COMPOSÉ COMME AGENT ANTI-VIEILLISSEMENT ET/OU ANTI-OXYDANT ET/OU COLORANT

(30) Priorität: 02.12.2021 DE 102021213722
(43) Veröffentlichungstag der Anmeldung: 09.10.2024
(73) Patentinhaber: Continental Reifen Deutschland GmbH, 30175 Hannover (DE)
(72) Erfinder: JACOB, Andreas, 30175 Hannover (DE); DAUER, David-Raphael, 30175 Hannover (DE); STROHMEIER, Julian, 30175 Hannover (DE)
(74) Vertreter: Continental Corporation
(86) Internationale Anmeldenummer: PCT/DE2022/200253
(87) Internationale Veröffentlichungsnummer: WO 2023/098953

(56) Entgegenhaltungen:
- EP-A1- 3 517 570
- DE-A1- 1 694 318
- ZHANG DEKUN ET AL: "Remote Enantioselective Desymmetrization of 9,9-Disubstituted 9,10-Dihydroacridines through Asymmetric Aromatic Aminations", ACS CATALYSIS, vol. 12, no. 23, 15 November 2022 (2022-11-15), US, pages 14609 - 14618, XP93016197, ISSN: 2155-5435, Retrieved from the Internet <URL:https://pubs.acs.org/doi/pdf/10.1021/acscatal.2c04975> DOI: 10.1021/acscatal.2c04975
- UNDERHILL JACK ET AL: "Dioxygen Splitting by a Tantalum(V) Complex Ligated by a Rigid, Redox Non-Innocent Pincer Ligand**", CHEMISTRY - A EUROPEAN JOURNAL, 29 November 2022 (2022-11-29), DE, XP093016191, ISSN: 0947-6539, Retrieved from the Internet <URL:https://onlinelibrary.wiley.com/doi/full-xml/10.1002/chem.202203266> DOI: 10.1002/chem.202203266
- ZHANG DEKUN ET AL: "Remote Enantioselective Desymmetrization of 9,9-Disubstituted 9,10-Dihydroacridines through Asymmetric Aromatic Aminations", ACS CATALYSIS, vol. 12, no. 23, 15 November 2022 (2022-11-15), US, pages 14609 - 14618, XP093016197, ISSN: 2155-5435, Retrieved from the Internet <URL:https://pubs.acs.org/doi/pdf/10.1021/acscatal.2c04975> DOI: 10.1021/acscatal.2c04975

## Beschreibung

Die Erfindung betrifft eine Verbindung, eine Kautschukmischung enthaltend die Verbindung, einen Fahrzeugreifen, der die Kautschukmischung in wenigstens einem Bauteil aufweist, Verfahren zur Herstellung der Verbindung sowie die Verwendung der Verbindung als Alterungsschutzmittel und/oder Antioxidationsmittel und/oder Farbstoff.

Es ist bekannt, dass in Fahrzeugreifen und technischen Gummiartikeln polymere Materialien, wie insbesondere Kautschuke, eingesetzt werden.

Naturkautschuk, synthetische Polymere (wie IR, BR, SSBR, ESBR, etc.) aber auch natürliche sowie synthetische Öle, Fette und Schmiermittel unterliegen bei längerer Lagerung und vor allem in der Zielanwendung, die oft bei höheren Temperaturen abläuft, Oxidationsreaktionen, die sich nachteilig auf die ursprünglichen gewünschten Eigenschaften auswirken. Je nach Art des Polymers werden die Polymerketten, bis hin zu einer Verflüssigung des Materials, verkürzt oder es kommt zur nachträglichen Härtung des Werkstoffes.

Alterungsschutzmittel tragen daher maßgeblich zur Langlebigkeit von Fahrzeugreifen und anderen technischen Gummiartikeln bei.

Bekannte Alterungsschutzmittel sind aromatische Amine, wie beispielsweise 6PPD (N-(1,3-Dimethylbutyl)-N'-phenyl-p-phenylendiamin), IPPD (N-Isopropyl-N'-phenyl-p-phenylendiamin) oder SPPD (N-(1-phenylethyl)-N'-phenyl-p-phenylendiamin).

Diese Moleküle können mit Sauerstoff oder Ozon oder gebildeten Radikalen, wie Alkyl-, Alkoxy- und Alkylperoxyradikalen, reagieren und diese somit abfangen und somit die Kautschuke etc. vor weiteren Oxidationsreaktionen schützen.

Nachteilig an dieser Substanzklasse ist allerdings der Verdacht, dass diese krebserregend sein könnten.

Alterungsschutzmittel, die insbesondere mit Ozon reagieren und dieses abfangen, werden auch als "Ozonschutzmittel" oder "Antiozonant" bezeichnet.

Der Erfindung liegt die Aufgabe zugrunde, eine neuartige Verbindung bereitzustellen, die insbesondere als Alterungsschutzmittel in Fahrzeugreifen oder anderen technischen Gummiartikeln verwendet werden kann, und zwar mit einem geringeren Gefahrenpotential bei hinreichender Löslichkeit in der jeweiligen Matrix, beispielsweise und insbesondere im Polymer. Hierdurch soll unter Verringerung der Gesundheitsschädlichkeit ein weiterhin optimaler Schutz vor Sauerstoff und Ozon gewährleistet werden sowie die Tendenz zum Ausblühen (engl. "Blooming") verhindert werden.

Gelöst wird die Aufgabe durch die erfindungsgemäße Verbindung gemäß Anspruch 1, die erfindungsgemäße Kautschukmischung enthaltend die Verbindung sowie den erfindungsgemäßen Fahrzeugreifen, der die erfindungsgemäße Kautschukmischung in wenigstens einem Bauteil aufweist.

Ferner wird die Aufgabe durch das erfindungsgemäße Verfahren zur Herstellung der erfindungsgemäßen Verbindung gelöst.

Die Verbindung gemäß Anspruch 1 weist die allgemeine Formel I) auf: 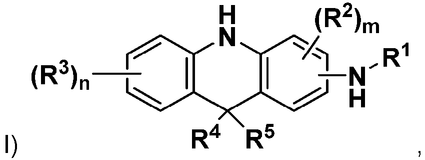
wobei R¹ ausgewählt ist aus der Gruppe bestehend aus Benzyl- und linearen, verzweigten und cyclischen aliphatischen C₃- bis C₁₂-Resten; und wobei R³ ausgewählt ist aus der Gruppe bestehend aus linearen, verzweigten und cyclischen aliphatischen C₁- bis C₁₂-Resten, sowie Aryl-Resten, Cyano-Resten, Halogen-Resten, wobei Fluor, Brom und Chlor bevorzugt sind, Ester-Resten, Keton-Resten, Ether-Resten und Thioether-Resten, und wobei n den Wert 0 oder 1 oder 2 oder 3 oder 4 annimmt, wobei die Reste R³ im Fall von n gleich 2 oder 3 oder 4 unabhängig voneinander gleich oder verschieden sind, und
wobei R² ausgewählt ist aus der Gruppe bestehend aus linearen, verzweigten und cyclischen aliphatischen C₁- bis C₁₂-Resten, sowie Aryl-Resten, Cyano-Resten, Halogen-Resten, wobei Fluor, Brom und Chlor bevorzugt sind, Ester-Resten, Keton-Resten, Ether-Resten und Thioether-Resten; und
wobei m den Wert 0 oder 1 oder 2 oder 3 annimmt, wobei die Reste R² im Fall von m gleich 2 oder 3 unabhängig voneinander gleich oder verschieden sind, und
wobei die Reste R⁴ und R⁵ unabhängig voneinander gleich oder verschieden sind und jeweils ausgewählt sind aus der Gruppe bestehend aus linearen, verzweigten und cyclischen aliphatischen C₁- bis C₁₂-Resten, sowie Aryl-Resten, Cyano-Resten, Halogen-Resten, wobei Fluor, Brom und Chlor bevorzugt sind, Ester-Resten, Keton-Resten, Ether-Resten und Thioether-Resten, wobei die linearen, verzweigten und cyclischen aliphatischen C₁- bis C₁₂-Reste sowie Aryl-Reste Substituenten tragen können.

Dem Fachmann ist klar, dass im Fall von n gleich 0 (null) oder 1 oder 2 oder 3 anstelle des R³ jeweils ein Wasserstoffatom an das entsprechende Kohlenstoffatom des Benzolringes gebunden ist. Ebenso sind im Fall von m gleich 0 oder 1 oder 2 alle übrigen freien Positionen am Benzolring des Gerüsts Wasserstoffatome. Dem Fachmann ist ebenfalls klar, dass die Darstellung der Verknüpfungen von (R²)ₘ und (R³)ₙ sowie R¹HN in den jeweiligen Benzolring des Gerüsts bedeutet, dass diese Gruppen jeweils an jeder Stelle am jeweiligen Benzolring angeordnet sein können, außer natürlich nicht jeweils zwei oder mehrere gleichzeitig an derselben Position, was schon aufgrund der Vierbindigkeit des Kohlenstoffatoms des Benzolrings ausgeschlossen wäre.

Bezeichnungen derart wie "C₃- bis C₁₂-Reste" bedeutet im Rahmen der vorliegenden Erfindung, dass Reste mit 3 bis 12 Kohlenstoffatomen gemeint sind. Unabhängig davon wird "C₁" als Bezeichnung der Position des höchstoxidierten Kohlenstoffatoms bzw. der höchsten Priorität nach der Cahn-Ingold-Prelog-Konvention (CIP) verwendet. Dem Fachmann ist im jeweiligen Kontext klar, was gemeint ist.

Die erfindungsgemäße Verbindung ist ein Acridin-Derivat und weist gegenüber bekannten Alterungsschutzmitteln auf Basis von Anilin (mögliches Spaltprodukt von 6PPD) ein geringeres Gefahrenpotential auf.

Die erfindungsgemäße Verbindung zeigt in Kautschukmischungen eine gegenüber dem 6PPD vergleichbare oder sogar verbesserte Alterungsschutzwirkung und ist somit als Ersatz von 6PPD geeignet, dessen Abbauprodukte extrem toxisch für den Silberlachs und damit wohl auch für andere aquatische Organismen sind.

Die erfindungsgemäße Verbindung weist zudem eine sehr gute Löslichkeit in Kautschukmischungen, insbesondere für Fahrzeugreifen und andere technische Gummiartikel, auf. Hierdurch wird ein Ausblühen dieser Verbindung, wie es von vielen Alterungsschutzmitteln bekannt ist, vermieden.

Von der Erfindung sind sämtliche vorteilhafte Ausgestaltungen, die sich unter anderem in den Patentansprüchen widerspiegeln, umfasst. Insbesondere sind von der Erfindung auch Ausgestaltungen umfasst, die sich durch Kombination unterschiedlicher Merkmale unterschiedlicher Abstufungen bei der Bevorzugung dieser Merkmale ergeben, sodass auch eine Kombination eines ersten als "bevorzugt" bezeichneten Merkmals oder im Rahmen einer vorteilhaften Ausführungsform beschriebenen Merkmals mit einem weiteren als z. B. "besonders bevorzugt" bezeichneten Merkmal von der Erfindung umfasst ist.

Die erfindungsgemäße Verbindung gemäß Formel I) ist besonders als Alterungsschutzmittel und/oder Ozonschutzmittel in Fahrzeugreifen und/oder anderen technischen Gummiartikeln, wie insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon, und/oder Ölen und/oder Schmierstoffen geeignet.

Die erfindungsgemäße Verbindung gemäß Formel I) ist besonders zur Herstellung eines Gummiartikels, insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon geeignet.

Zu Verwendung der Verbindung gemäß Formel I) in den genannten Artikeln oder Stoffen wird diese in einer Zusammensetzung verwendet und in dieser eingemischt verwendet.

Bei Fahrzeugreifen oder anderen technischen Gummiartikeln ist dies insbesondere eine Kautschukmischung.

Ein weiterer Gegenstand der Erfindung ist die Verwendung der erfindungsgemäßen Verbindung gemäß Formel I) in Ölen, Schmierstoffen, wie insbesondere Treib- oder Betriebsstoffen für Motoren. Insbesondere kann die erfindungsgemäße Verbindung somit in Motoren verwendet werden.

Ein weiterer Gegenstand der Erfindung ist die Verwendung der erfindungsgemäßen Verbindung gemäß Formel I) als Farbstoff in Fasern und/oder Polymeren und/oder Papier und/oder in (Streich-)Farben und Lacken.

Bevorzugt ist in Formel I) n gleich 0 (null).

Bevorzugt ist in Formel I) m gleich 0 (null).

Es ist bevorzugt, dass R¹ über ein tertiäres Kohlenstoffatom an das Stickstoffatom (N) gebunden ist. Damit ist das C₁-Atom bevorzugt ein tertiäres Kohlenstoffatom. Unter dem Begriff "tertiäres Kohlenstoffatom" ist im Rahmen der vorliegenden Erfindung ein Kohlenstoffatom zu verstehen, welches an nur ein Wasserstoffatom gebunden ist.

Hiermit ergibt sich eine besonders gute Löslichkeit der erfindungsgemäßen Verbindung in Kautschukmischungen, insbesondere für Fahrzeugreifen und andere technische Gummiartikel, sowie eine optimierte Reaktivität im Zusammenhang mit den für den Alterungsschutz relevanten Mechanismen.

Besonders bevorzugt ist R¹ ein verzweigter Alkyl-Rest mit 3 bis 12 Kohlenstoffatomen, wiederum bevorzugt 3 bis 8 Kohlenstoffatomen. Hierbei ist bevorzugt zumindest eine Verzweigung am C₁-Atom vorhanden, also an dem an das Stickstoffatom (N) gebundene Kohlenstoffatom, womit das C₁-Atom ein tertiäres Kohlenstoffatom ist.

Hiermit ergibt sich eine besonders gute Löslichkeit der erfindungsgemäßen Verbindung in Kautschukmischungen, insbesondere für Fahrzeugreifen und andere technische Gummiartikel, sowie eine optimierte Reaktivität im Zusammenhang mit den für den Alterungsschutz relevanten Mechanismen.

Ganz besonders bevorzugt ist R¹ ausgewählt aus 1,3-Dimethylbutyl- und Cyclohexyl-Resten, wiederum ganz besonders bevorzugt ist R¹ ein 1,3-Dimethylbutyl-Rest.

Hiermit ergibt sich eine besonders gute Löslichkeit der erfindungsgemäßen Verbindung in Kautschukmischungen, insbesondere für Fahrzeugreifen und andere technische Gummiartikel.

Die linearen, verzweigten und cyclischen aliphatischen C₁- bis C₁₂-Reste sowie Aryl-Reste können Substituenten tragen.

Bevorzugt sind die Reste R⁴ und R⁵ jeweils ausgewählt aus der Gruppe bestehend aus nicht-substituierten, linearen, verzweigten und cyclischen aliphatischen C₁- bis C₁₂-Resten, und Aryl-Resten. Besonders bevorzugt sind die Reste R⁴ und R⁵ dabei ausgewählt aus der Gruppe bestehend aus Methyl-Resten, n-Butyl-Resten und Phenyl-Resten.

Diese Reste sind besonders gut über die Synthese ausgehend von den entsprechenden Lithiumverbindungen zugänglich.

Ganz besonders bevorzugt sind die Reste R⁴ und R⁵ jeweils ausgewählt aus der Gruppe bestehend aus linearen C₁- bis C₁₂-Resten, besonders bevorzugt bestehend aus linearen C₁- bis C₆-Resten, wobei Methyl-Reste ganz besonders bevorzugt sind.

Hiermit ergibt sich eine besonders gute Löslichkeit der erfindungsgemäßen Verbindung in Kautschukmischungen, insbesondere für Fahrzeugreifen und andere technische Gummiartikel, sowie eine optimierte Reaktivität im Zusammenhang mit den für den Alterungsschutz relevanten Mechanismen.

Ferner weist die erfindungsgemäße Verbindung hiermit eine sehr gute Stabilität, insbesondere gegenüber der Oxidation zum Acridin, auf.

Gemäß besonders bevorzugter Ausführungsformen der Erfindung sind die Reste R⁴ und R⁵ gleich.

Ganz besonders bevorzugt sind sowohl R⁴ als auch R⁵ jeweils ein Methyl-Rest. Hiermit ergibt sich eine optimale Löslichkeit der erfindungsgemäßen Verbindung in Kautschukmischungen, insbesondere für Fahrzeugreifen und andere technische Gummiartikel.

Ferner weist die erfindungsgemäße Verbindung hiermit eine sehr gute Stabilität, insbesondere gegenüber der Oxidation zum Acridin, auf.

In einer bevorzugten Ausführungsform weist die erfindungsgemäße Verbindung gemäß Formel I) die Struktur gemäß Formel II) auf:

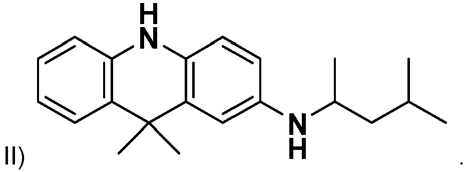

Mit der Verbindung gemäß Formel II) wird die der Erfindung zugrunde liegende Aufgabe besonders gut gelöst.

Mit der Verbindung gemäß Formel II) kann, insbesondere in Polymeren, sogar eine weitere Verbesserung des Schutzes vor Oxidation und somit Alterung erzielt werden. Gleichzeitig ist die Verbindung gemäß Formel II) deutlich weniger gesundheitsschädlich als z. B. 6PPD oder andere Vertreter dieser Substanzklasse, wie einleitend aufgeführt.

Im Vergleich zu 6PPD ist die Verbindung gemäß Formel II) somit ein besseres und gleichzeitig gesundheits- und umweltfreundlicheres Alterungsschutzmittel.

Gemäß weiterer bevorzugter Ausführungsformen unterscheiden sich die Reste R⁴ und R⁵ voneinander. Besonders bevorzugt ist hierbei einer der Reste R⁴ oder R⁵ ein Aryl-Rest, insbesondere ein Phenyl-Rest, und der andere ausgewählt aus der Gruppe bestehend aus linearen C₁- bis C₁₂-Resten, besonders bevorzugt linearen C₁- bis C₆-Resten, und ganz besonders bevorzugt ein Methylrest.

In einer weiteren bevorzugten Ausführungsform weist die erfindungsgemäße Verbindung gemäß Formel I) die Struktur gemäß Formel III) auf:

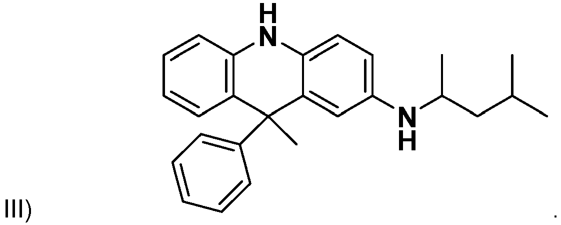

Ein weiterer Gegenstand der Erfindung ist wie oben ausgeführt eine Kautschukmischung.

Die erfindungsgemäße Kautschukmischung enthält die Verbindung gemäß Formel I). Bei der erfindungsgemäßen Kautschukmischung kann es sich prinzipiell um jede Kautschukmischung handeln, insbesondere in der die neuartige erfindungsgemäße Verbindung gemäß Formel I) als Alterungsschutzmittel und/oder Ozonschutzmittel bei geringerer Toxizität wirkt.

Die erfindungsgemäße Kautschukmischung enthält wenigstens einen Kautschuk.

Bevorzugt enthält die erfindungsgemäße Kautschukmischung 0,1 bis 10 phr, besonders bevorzugt 0,1 bis 7 phr, ganz besonders bevorzugt 1 bis 6 phr, der Verbindung gemäß Formel I).

Die in dieser Schrift verwendete Angabe phr (parts per hundred parts of rubber by weight) ist die in der Kautschukindustrie übliche Mengenangabe für Mischungsrezepturen. Die Dosierung der Gewichtsteile der einzelnen Substanzen wird in dieser Schrift auf 100 Gewichtsteile der gesamten Masse aller in der Mischung vorhandenen hochmolekularen (M_{w} größer als 20.000 g/mol) Kautschuke bezogen.

Gemäß vorteilhafter Ausführungsformen der Erfindung enthält die erfindungsgemäße Kautschukmischung wenigstens einen Dienkautschuk.

Die Kautschukmischung kann somit einen Dienkautschuk oder ein Gemisch von zwei oder mehreren verschiedenen Dienkautschuken enthalten.

Als Dienkautschuke werden Kautschuke bezeichnet, die durch Polymerisation oder Copolymerisation von Dienen und/oder Cycloalkenen entstehen und somit entweder in der Hauptkette oder in den Seitengruppen C=C-Doppelbindungen aufweisen.

Der Dienkautschuk ist dabei bevorzugt ausgewählt aus der Gruppe bestehend aus natürlichem Polyisopren (NR), synthetischem Polyisopren (IR), epoxidiertem Polyisopren (ENR), Butadien-Kautschuk (BR), Butadien-Isopren-Kautschuk, lösungspolymerisiertem Styrol-Butadien-Kautschuk (SSBR), emulsionspolymerisiertem Styrol-Butadien-Kautschuk (ESBR), Styrol-Isopren-Kautschuk, Flüssigkautschuken mit einem Molekulargewicht M_{w} von größer als 20000 g/mol, Halobutyl-Kautschuk, Polynorbornen, Isopren-Isobutylen-Copolymer, Ethylen-Propylen-Dien-Kautschuk, Nitril-Kautschuk, Chloropren-Kautschuk, Acrylat-Kautschuk, Fluor-Kautschuk, Silikon-Kautschuk, Polysulfid-Kautschuk, Epichlorhydrin-Kautschuk, Styrol-Isopren-Butadien-Terpolymer, hydriertem Acrylnitrilbutadien-Kautschuk und hydriertem Styrol-Butadien-Kautschuk.

Insbesondere Nitrilkautschuk, hydrierter Acrylnitrilbutadienkautschuk, Chloroprenkautschuk, Butylkautschuk, Halobutylkautschuk und/oder Ethylen-Propylen-Dien-Kautschuk kommen bei der Herstellung von technischen Gummiartikeln, wie Gurte, Riemen und Schläuche, und/oder Schuhsohlen zum Einsatz. Dabei finden die dem Fachmann für diese Kautschuke bekannten - im Hinblick auf Füllstoffe, Weichmacher, Vulkanisationssysteme und Zuschlagstoffe besonderen - Mischungszusammensetzungen bevorzugte Anwendung.

Bei dem natürlichen und/oder synthetischen Polyisopren sämtlicher Ausführungsformen kann es sich sowohl um cis-1,4-Polyisopren als auch um 3,4-Polyisopren handeln. Bevorzugt ist allerdings die Verwendung von cis-1,4-Polyisoprenen mit einem cis-1,4 Anteil > 90 Gew.-%. Zum einen kann solch ein Polyisopren durch stereospezifische Polymerisation in Lösung mit Ziegler-Natta-Katalysatoren oder unter Verwendung von fein verteilten Lithiumalkylen erhalten werden. Zum anderen handelt es sich bei Naturkautschuk (NR) um ein solches cis-1,4 Polyisopren, bei welchem der cis-1,4-Anteil im Naturkautschuk größer 99 Gew.-% ist.

Ferner ist auch ein Gemisch eines oder mehrerer natürlicher Polyisoprene mit einem oder mehreren synthetischen Polyisopren(en) denkbar.

Im Rahmen der vorliegenden Erfindung ist unter dem Begriff "Naturkautschuk" natürlich vorkommender Kautschuk zu verstehen, der von Hevea Gummibäumen und "Nicht-Hevea" Quellen gewonnen werden kann. Nicht-Hevea Quellen sind beispielsweise Guayule Sträucher und Löwenzahn wie beispielsweise TKS (Taraxacum kok-saghyz; Russischer Löwenzahn).

Falls in der erfindungsgemäßen Kautschukmischung Butadien-Kautschuk (= BR, Polybutadien) enthalten ist, kann es sich um alle dem Fachmann bekannten Typen handeln. Darunter fallen u.a. die sogenannten high-cis- und low-cis-Typen, wobei Polybutadien mit einem cis-Anteil größer oder gleich 90 Gew.-% als high-cis-Typ und Polybutadien mit einem cis-Anteil kleiner als 90 Gew.-% als low-cis-Typ bezeichnet wird. Ein low-cis-Polybutadien ist z.B. Li-BR (Lithium-katalysierter Butadien-Kautschuk) mit einem cis-Anteil von 20 bis 50 Gew.-%. Mit einem high-cis BR werden besonders gute Eigenschaften sowie eine niedrige Hysterese der Kautschukmischung erzielt.

Das oder die eingesetzte(n) Polybutadiene kann/können mit Modifizierungen und Funktionalisierungen endgruppenmodifiziert und/oder entlang der Polymerketten funktionalisiert sein. Bei der Modifizierung kann es sich um solche mit Hydroxy-Gruppen und/oder Ethoxy-Gruppen und/oder Epoxy-Gruppen und/oder Siloxan-Gruppen und/oder Amino-Gruppen und/oder Aminosiloxan und/oder Carboxy-Gruppen und/oder Phthalocyanin-Gruppen und/oder Silan-Sulfid-Gruppen handeln. Es kommen aber auch weitere, dem Fachmann bekannte, Modifizierungen, auch als Funktionalisierungen bezeichnet, in Frage. Bestandteil solcher Funktionalisierungen können Metallatome sein.

Für den Fall, dass wenigstens ein Styrol-Butadien-Kautschuk (Styrol-Butadien-Copolymer) in der Kautschukmischung enthalten ist, kann es sich sowohl um lösungspolymerisierten Styrol-Butadien-Kautschuk (SSBR) als auch um emulsionspolymerisierten Styrol-Butadien-Kautschuk (ESBR) handeln, wobei auch ein Gemisch aus wenigstens einem SSBR und wenigstens einem ESBR eingesetzt werden kann. Die Begriffe "Styrol-Butadien-Kautschuk" und "Styrol-Butadien-Copolymer" werden im Rahmen der vorliegenden Erfindung synonym verwendet.

Das eingesetzte Styrol-Butadien-Copolymer kann mit den oben beim Polybutadien genannten Modifizierungen und Funktionalisierungen endgruppenmodifiziert und/oder entlang der Polymerketten funktionalisiert sein.

Vorzugsweise ist der wenigstens eine Dienkautschuk ausgewählt aus der Gruppe bestehend aus natürlichem Polyisopren (NR, Naturkautschuk), synthetischem Polyisopren (IR), Butadien-Kautschuk (BR), lösungspolymerisiertem Styrol-Butadien-Kautschuk (SSBR), emulsionspolymerisiertem Styrol-Butadien-Kautschuk (ESBR), Butylkautschuk (IIR) und Halobutylkautschuk.

Gemäß einer besonders bevorzugten Ausführungsform der Erfindung ist der wenigstens eine Dienkautschuk ausgewählt aus der Gruppe bestehend aus natürlichem Polyisopren (NR), synthetischem Polyisopren (IR), Butadien-Kautschuk (BR), lösungspolymerisiertem Styrol-Butadien-Kautschuk (SSBR) und emulsionspolymerisiertem Styrol-Butadien-Kautschuk (ESBR).

Gemäß einer besonders vorteilhaften Ausführungsform der Erfindung enthält die Kautschukmischung wenigstens ein natürliches Polyisopren (NR) und zwar bevorzugt in Mengen von 50 bis 100 phr, und gemäß einer besonders vorteilhaften Ausführungsform der Erfindung 80 bis 100 phr, ganz besonders bevorzugt 95 bis 100 phr, wiederum bevorzugt 100 phr. Eine derartige Kautschukmischung zeigt insbesondere optimierte Reißeigenschaften und Abriebeigenschaften bei einer guten Verarbeitbarkeit und Reversionsstabilität.

Für den Fall, dass die Kautschukmischung weniger als 100 phr NR enthält, enthält sie bevorzugt als weiteren Kautschuk wenigstens einen Dienkautschuk, der ausgewählt ist aus der Gruppe bestehend aus synthetischem Polyisopren (IR), Butadien-Kautschuk (BR), lösungspolymerisiertem Styrol-Butadien-Kautschuk (SSBR) und emulsionspolymerisiertem Styrol-Butadien-Kautschuk (ESBR).

Gemäß einer weiteren besonders vorteilhaften Ausführungsform der Erfindung enthält die Kautschukmischung wenigstens ein natürliches Polyisopren (NR) und zwar bevorzugt in Mengen von 5 bis 55 phr, und gemäß einer besonders vorteilhaften Ausführungsform der Erfindung 5 bis 25 phr, ganz besonders bevorzugt 5 bis 20 phr. Eine derartige Kautschukmischung zeigt insbesondere eine gute Verarbeitbarkeit und Reversionsstabilität sowie optimierte Reißeigenschaften und ein optimales Rollwiderstandsverhalten.

Gemäß einer weiteren besonders vorteilhaften Ausführungsform der Erfindung enthält die Kautschukmischung wenigstens ein Polybutadien (BR, Butadienkautschuk) und zwar bevorzugt in Mengen von 10 bis 80 phr, besonders bevorzugt 10 bis 50 phr, und gemäß einer besonders vorteilhaften Ausführungsform der Erfindung 15 bis 40 phr. Hiermit werden besonders gute Reiß- und Abriebeigenschaften der erfindungsgemäßen Kautschukmischung und ein optimales Bremsverhalten erzielt.

Gemäß einer weiteren besonders vorteilhaften Ausführungsform der Erfindung enthält die Kautschukmischung wenigstens einen lösungspolymerisierten Styrol-Butadien-Kautschuk (SSBR) und zwar bevorzugt in Mengen von 10 bis 80 phr, besonders bevorzugt 30 bis 80 phr, und gemäß einer besonders vorteilhaften Ausführungsform der Erfindung 50 bis 70 phr. Hiermit werden besonders gute Rollwiderstandseigenschaften der erfindungsgemäßen Kautschukmischung erzielt. Gemäß besonders vorteilhaften Ausführungsformen der Erfindung wird SSBR in Kombination mit wenigstens einem weiteren Kautschuk eingesetzt, um ein optimales und ausbalanciertes Eigenschaftsprofil zu erzielen.

Bevorzugt enthält die Kautschukmischung wenigstens einen Füllstoff, bevorzugt in Mengen von 30 bis 500 phr, besonders bevorzugt 50 bis 400 phr, wiederum bevorzugt 80 bis 300 phr.

Gemäß vorteilhafter Ausführungsformen der Erfindung ist der Füllstoff ein verstärkender Füllstoff, der bevorzugt ausgewählt ist aus der Gruppe bestehend aus Rußen und Siliciumdioxid.

Als Ruße kommen alle dem Fachmann bekannten Rußtypen in Frage. Bevorzugt ist der Ruß ausgewählt aus Industrierußen und Pyrolyse-Rußen, wobei Industrieruße weiter bevorzugt sind.

Bevorzugt hat der Ruß eine Jodzahl, gemäß ASTM D 1510, die auch als Jodadsorptionszahl bezeichnet wird, zwischen 30 und 250 g/kg, bevorzugt 30 bis 180 g/kg, besonders bevorzugt 40 bis 180 g/kg, und ganz besonders bevorzugt 40 bis 130 g/kg, und eine DBP-Zahl gemäß ASTM D 2414 von 30 bis 200 ml/100 g, bevorzugt 70 bis 200 ml/100g, besonders bevorzugt 90 bis 200 ml/100g.

Die DBP-Zahl gemäß ASTM D 2414 bestimmt das spezifische Absorptionsvolumen eines Rußes oder eines hellen Füllstoffes mittels Dibutylphthalat.

Die Verwendung eines solchen Rußtyps in der Kautschukmischung, insbesondere für Fahrzeugreifen, gewährleistet einen bestmöglichen Kompromiss aus Abriebwiderstand und Wärmeaufbau, der wiederum den ökologisch relevanten Rollwiderstand beeinflusst.

Besonders geeignet und bevorzugt ist dabei ein Ruß mit einer Jodadsorptionszahl zwischen 80 und 110 g/kg und einer DBP-Zahl von 100 bis 130 ml/100g, wie insbesondere Rußes des Types N339.

Das Siliciumdioxid ist bevorzugt amorphes Siliciumdioxid, beispielsweise gefällte Kieselsäure, die auch als gefälltes Siliciumdioxid bezeichnet wird. Alternativ kann aber beispielsweise auch pyrogenes Siliciumdioxid eingesetzt werden. Besonders bevorzugt ist es allerdings, wenn eine fein verteilte, gefällte Kieselsäure verwendet wird, die eine Stickstoff-Oberfläche (BET-Oberfläche) (gemäß DIN ISO 9277 und DIN 66132) von 35 bis 400 m²/g, bevorzugt von 35 bis 350 m²/g, besonders bevorzugt von 85 bis 320 m²/g und ganz besonders bevorzugt von 120 bis 235 m²/g, und eine CTAB-Oberfläche (gemäß ASTM D 3765) von 30 bis 400 m²/g, bevorzugt von 30 bis 330 m²/g, besonders bevorzugt von 80 bis 300 m²/g und ganz besonders bevorzugt von 115 bis 200 m²/g, aufweist. Derartige Kieselsäuren führen z. B. in Kautschukmischungen für Reifenlaufstreifen zu besonders guten physikalischen Eigenschaften der Vulkanisate. Außerdem können sich dabei Vorteile in der Mischungsverarbeitung durch eine Verringerung der Mischzeit bei gleichbleibenden Produkteigenschaften ergeben, die zu einer verbesserten Produktivität führen. Als Kieselsäuren können somit z. B. sowohl jene des Typs Ultrasil^{®} VN3 (Handelsname) der Firma Evonik als auch hoch dispergierbare Kieselsäuren, so genannte HD-Kieselsäuren (z. B. Zeosil^{®} 1165 MP der Firma Solvay), zum Einsatz kommen.

Gemäß besonders vorteilhafter Ausführungsformen der Erfindung enthält die Kautschukmischung wenigstens eine Kieselsäure als Füllstoff, bevorzugt in Mengen von 30 bis 500 phr, besonders bevorzugt 50 bis 400 phr, wiederum bevorzugt 80 bis 300 phr.

In diesen Mengen ist Kieselsäure insbesondere als alleiniger oder als Hauptfüllstoff (mehr als 50 Gew.-% bezogen auf die Gesamtfüllstoffmenge) enthalten.

Gemäß weiterer vorteilhafter Ausführungsformen der Erfindung enthält die Kautschukmischung wenigstens eine Kieselsäure als weiteren Füllstoff, und zwar bevorzugt in Mengen von 5 bis 100 phr, besonders bevorzugt 5 bis 80 phr, wiederum bevorzugt 10 bis 60 phr.

In diesen Mengen ist Kieselsäure insbesondere als weiterer Füllstoff zusätzlich zu einem anderen Hauptfüllstoff, wie insbesondere einem Ruß, enthalten.

Die Begriffe "Kieselsäure" und "Silika" werden im Rahmen der vorliegenden Erfindung synonym verwendet.

Gemäß besonders vorteilhafter Ausführungsformen der Erfindung enthält die erfindungsgemäße Kautschukmischung 0,1 bis 60 phr, bevorzugt 3 bis 40 phr, besonders bevorzugt 5 bis 30 phr, ganz besonders bevorzugt 5 bis 15 phr, wenigstens eines Rußes. In diesen Mengen ist Ruß insbesondere als weiterer Füllstoff zusätzlich zu einem Hauptfüllstoff, wie insbesondere Kieselsäure, enthalten.

Gemäß weiterer vorteilhafter Ausführungsformen der Erfindung enthält die erfindungsgemäße Kautschukmischung 30 bis 300 phr, bevorzugt 30 bis 200 phr, besonders bevorzugt 40 bis 100 phr wenigstens eines Rußes. In diesen Mengen ist Ruß als alleiniger oder als Hauptfüllstoff und dabei ggf. in Kombination mit Kieselsäure in den oben genannten geringeren Mengen enthalten.

Gemäß einer besonders vorteilhaften Ausführungsform der Erfindung enthält die Kautschukmischung 5 bis 60 phr, besonders bevorzugt 5 bis 40 phr, wenigstens eines Rußes und 50 bis 300 phr, bevorzugt 80 bis 200 phr wenigstens einer Kieselsäure.

Die Kautschukmischung kann zudem weitere Füllstoffe enthalten, die verstärkend wirken oder nicht verstärkend wirken.

Zu den weiteren (nicht verstärkenden) Füllstoffen zählen im Rahmen der vorliegenden Erfindung Alumosilicate, Kaolin, Kreide, Stärke, Magnesiumoxid, Titandioxid oder Kautschukgele sowie Fasern (wie zum Beispiel Aramidfasern, Glasfasern, Carbonfasern, Cellulosefasern).

Weitere ggf. verstärkende Füllstoffe sind z.B. Kohlenstoffnanoröhrchen (carbon nanotubes (CNT) inklusive diskreter CNTs, sogenannte hollow carbon fibers (HCF) und modifizierte CNT enthaltend eine oder mehrere funktionelle Gruppen, wie Hydroxy-, Carboxy und Carbonyl-Gruppen), Graphit und Graphene und sogenannte "carbon-silica dual-phase filler".

Zinkoxid gehört im Rahmen der vorliegenden Erfindung nicht zu den Füllstoffen.

Des Weiteren kann die Kautschukmischung übliche Zusatzstoffe in üblichen Gewichtsteilen enthalten, die bei deren Herstellung bevorzugt in wenigstens einer Grundmischstufe zugegeben werden. Zu diesen Zusatzstoffen zählen
a) im Stand der Technik bekannte Alterungsschutzmittel,
   wie z. B. p-Phenylendiamine, wie
   N-Phenyl-N'-(1,3-dimethylbutyl)-p-phenylendiamin (6PPD),
   N,N'-Diphenyl-p-phenylendiamin (DPPD),
   N-(1-phenylethyl)-N'-phenyl-p-phenylendiamin (SPPD),
   N,N'-Ditolyl-p-phenylendiamin (DTPD),
   N-(1,4-dimethylpentyl)-N'-phenyl-p-phenylendiamin (7PPD),
   N-Isopropyl-N'-phenyl-p-phenylendiamin (IPPD),
   oder Dihydrochinoline, wie 2,2,4-Trimethyl-1,2-dihydrochinolin (TMQ),
b) Aktivatoren, wie z. B. Zinkoxid und Fettsäuren (z. B. Stearinsäure) und/oder sonstige Aktivatoren, wie Zinkkomplexe wie z.B. Zinkethylhexanoat,
c) Aktivatoren und/oder Agenzien für die Anbindung von Füllstoffen, insbesondere Ruß oder Kieselsäure, wie beispielsweise S-(3-Aminopropyl)Thioschwefelsäure und/oder deren Metallsalze (Anbindung an Ruß) sowie Silan-Kupplungsagenzien (Anbindung an Siliciumdioxid, insbesondere Kieselsäure),
d) Ozonschutzwachse,
e) Harze, insbesondere Klebharze,
f) Mastikationshilfsmittel, wie z. B. 2,2'-Dibenzamidodiphenyldisulfid (DBD) und
g) Prozesshilfsmittel, wie insbesondere Fettsäureester und Metallseifen, wie z.B. Zinkseifen und/oder Calciumseifen
h) Weichmacher, wie insbesondere wie aromatische, naphthenische oder paraffinische Mineralölweichmacher, wie z.B. MES (mild extraction solvate) oder RAE (Residual Aromatic Extract) oder TDAE (treated distillate aromatic extract), oder Rubber-to-Liquid-Öle (RTL) oder Biomass-to-Liquid-Öle (BTL) bevorzugt mit einem Gehalt an polycyclischen Aromaten von weniger als 3 Gew.-% gemäß Methode IP 346 oder Triglyceride, wie z. B. Rapsöl, oder Faktisse oder Kohlenwasserstoffharze oder Flüssig-Polymere, deren mittleres Molekulargewicht (Bestimmung per GPC = gel permeation chromatography, in Anlehnung an BS ISO 11344:2004) zwischen 500 und 20000 g/mol liegt.

Bei der Verwendung von Mineralöl ist dieses bevorzugt ausgewählt aus der Gruppe, bestehend aus DAE (Destillated Aromatic Extracts), RAE (Residual Aromatic Extract), TDAE (Treated Destillated Aromatic Extracts), MES (Mild Extracted Solvents) und naphthenischen Ölen.

Gemäß besonders vorteilhafter Ausführungsformen enthält die erfindungsgemäße Kautschukmischung neben der erfindungsgemäßen Verbindung gemäß Formel I) keine Alterungsschutzmittel aus der Gruppe der p-Phenylendiamine, insbesondere solche unter obiger Auflistung a). Insbesondere enthält die erfindungsgemäße Kautschukmischung gemäß einer besonders bevorzugten Ausführungsform 0 bis 0,1 phr, insbesondere 0 phr, an weiteren Alterungsschutzmitteln auf Basis von p-Phenylendiaminen, die ausgewählt sind aus der Gruppe enthaltend, bevorzugt bestehend aus, N-Phenyl-N'-(1,3-dimethylbutyl)-p-phenylendiamin (6PPD), N-(1-phenylethyl)-N'-phenyl-p-phenylendiamin (SPPD), N,N'-Diphenyl-p-phenylendiamin (DPPD), N,N'-Ditolyl-p-phenylendiamin (DTPD), N-Isopropyl-N'-phenyl-p-phenylendiamin (IPPD), N-(1,4-dimethylpentyl)-N'-phenyl-p-phenylendiamin (7PPD).

Mit den bevorzugt sehr geringen Mengen von 0 bis 0,1 phr bzw. besonders bevorzugt 0 phr an p-Phenylendiaminen, und der erfindungsgemäß enthaltenen Verbindung gemäß Formel I) ist es möglich eine vergleichbare Schutzwirkung bei geringerer Toxizität zu erzielen. Hierbei ersetzt die erfindungsgemäße Verbindung gemäß Formel I) die genannten im Stand der Technik bekannten p-Phenylendiamine.

Gemäß weiterer vorteilhafter Ausführungsformen der Erfindung ist noch wenigstens ein weiteres der genannten p-Phenylendiamin-Alterungsschutzmittel enthalten, sodass die erfindungsgemäße Verbindung die im Stand der Technik bekannten p-Phenylendiamine nur teilweise ersetzt. Hierdurch wird der erfindungsgemäße Vorteil auch erzielt, nur nicht in optimalem Ausmaß.

Alterungsschutzmittel auf Basis von Dihydrochinolin, wie TMQ, sind gemäß vorteilhafter Ausführungsformen neben der erfindungsgemäßen Verbindung gemäß Formel I) in der Kautschukmischung enthalten. Die Menge an enthaltenen Dihydrochinolinen, wie insbesondere TMQ, beträgt bevorzugt 0,1 bis 3, insbesondere 0,5 bis 1,5 phr.

Ozonschutzwachse (obige Gruppe d) werden separat betrachtet und sind gemäß bevorzugter Ausführungsformen der Erfindung in der Kautschukmischung enthalten, unabhängig davon ob zusätzliche Alterungsschutzmittel a) enthalten sind.

Bei den Silan-Kupplungsagenzien kann es sich um alle dem Fachmann bekannten Typen handeln.

Ferner können ein oder mehrere verschiedene Silan-Kupplungsagenzien in Kombination miteinander eingesetzt werden. Die Kautschukmischung kann somit ein Gemisch verschiedener Silane enthalten.

Die Silan-Kupplungsagenzien reagieren mit den oberflächlichen Silanolgruppen des Siliciumdioxids, insbesondere der Kieselsäure, oder anderen polaren Gruppen während des Mischens des Kautschuks bzw. der Kautschukmischung (in situ) oder bereits vor der Zugabe des Füllstoffes zum Kautschuk im Sinne einer Vorbehandlung (Vormodifizierung).

Aus dem Stand der Technik bekannten Kupplungsagenzien sind bifunktionelle Organosilane, die am Siliciumatom mindestens eine Alkoxy-, Cycloalkoxy- oder Phenoxygruppe als Abgangsgruppe besitzen und die als andere Funktionalität eine Gruppe aufweisen, die gegebenenfalls nach Spaltung eine chemische Reaktion mit den Doppelbindungen des Polymers eingehen kann. Bei der letztgenannten Gruppe kann es sich z. B. um die folgenden chemischen Gruppen handeln: -SCN, -SH, -NH₂ oder -Sₓ- (mit x = 2 bis 8).

So können als Silan-Kupplungsagenzien z. B. 3-Mercaptopropyltriethoxysilan, 3-Thiocyanato-propyltrimethoxysilan oder 3,3'-Bis(triethoxysilylpropyl)polysulfide mit 2 bis 8 Schwefelatomen, wie z. B. 3,3'-Bis(triethoxysilylpropyl)tetrasulfid (TESPT), das entsprechende Disulfid (TESPD) oder auch Gemische aus den Sulfiden mit 1 bis 8 Schwefelatomen mit unterschiedlichen Gehalten an den verschiedenen Sulfiden, verwendet werden. TESPT kann dabei beispielsweise auch als Gemisch mit Industrieruß (Handelsname X50S^{®} der Firma Evonik) zugesetzt werden.

Auch geblockte Mercaptosilane, wie sie z. B. aus der WO 99/09036 bekannt sind, können als Silan-Kupplungsagens eingesetzt werden. Auch Silane, wie sie in der WO 2008/083241 A1, der WO 2008/083242 A1, der WO 2008/083243 A1 und der WO 2008/083244 A1 beschrieben sind, können eingesetzt werden. Verwendbar sind z. B. Silane, die unter dem Namen NXT in verschiedenen Varianten von der Firma Momentive, USA, wie insbesondere 3-Octanoylthio-1-propyltriethoxysilan, oder solche, die unter dem Namen VP Si 363^{®} von der Firma Evonik Industries vertrieben werden.

Der Mengenanteil der Gesamtmenge an weiteren Zusatzstoffen beträgt bevorzugt 3 bis 150 phr, besonders bevorzugt 3 bis 100 phr und ganz besonders bevorzugt 5 bis 80 phr.

Im Gesamtmengenanteil der weiteren Zusatzstoffe kann Zinkoxid (ZnO) in den oben genannten Mengen enthalten sein.

Hierbei kann es sich um alle dem Fachmann bekannten Typen an Zinkoxid handeln, wie z.B. ZnO-Granulat oder -Pulver. Das herkömmlicherweise verwendete Zinkoxid weist in der Regel eine BET-Oberfläche von weniger als 10 m²/g auf. Es kann aber auch ein Zinkoxid mit einer BET-Oberfläche von 10 bis 100 m²/g, wie z.B. so genannte "nano-Zinkoxide", verwendet werden.

Die erfindungsgemäße Kautschukmischung wird bevorzugt vulkanisiert verwendet, insbesondere in Fahrzeugreifen oder anderen vulkanisierten technischen Gummiartikeln.

Die Begriffe "vulkanisiert" und "vernetzt" werden im Rahmen der vorliegenden Erfindung synonym verwendet.

Die Vulkanisation der erfindungsgemäßen Kautschukmischung wird bevorzugt in Anwesenheit von Schwefel und/oder Schwefelspendern mit Hilfe von Vulkanisationsbeschleunigern durchgeführt, wobei einige Vulkanisationsbeschleuniger zugleich als Schwefelspender wirken können. Dabei ist der Beschleuniger ausgewählt aus der Gruppe bestehend aus Thiazolbeschleunigern, Mercaptobeschleunigern, Sulfenamidbeschleunigern, Thiocarbamatbeschleunigern, Thiurambeschleunigern, Thiophosphatbeschleunigern, Thioharnstoffbeschleunigern, Xanthogenat-Beschleunigern und Guanidin-Beschleunigern. Bevorzugt ist die Verwendung eines Sulfenamidbeschleunigers, der ausgewählt ist aus der Gruppe bestehend aus N-Cyclohexyl-2-benzothiazolsufenamid (CBS), N,N-Dicyclohexylbenzothiazol-2-sulfenamid (DCBS), Benzothiazyl-2-sulfenmorpholid (MBS), N-tert-Butyl-2-benzothiazylsulfenamid (TBBS) und Guanidin-Beschleunigern wie Diphenylguanidin (DPG).

Als schwefelspendende Substanz können dabei alle dem Fachmann bekannten schwefelspendenden Substanzen verwendet werden.

Außerdem können in der Kautschukmischung Vulkanisationsverzögerer vorhanden sein.

Die Herstellung der Kautschukmischung erfolgt bevorzugt ansonsten nach dem in der Kautschukindustrie üblichen Verfahren, bei dem zunächst in ein oder mehreren Mischstufen eine Grundmischung mit allen Bestandteilen außer dem Vulkanisationssystem (z. B. Schwefel und vulkanisationsbeeinflussende Substanzen) hergestellt wird. Durch Zugabe des Vulkanisationssystems in einer letzten Mischstufe wird die Fertigmischung erzeugt.

Die Fertigmischung wird z.B. durch einen Extrusionsvorgang oder Kalandrieren weiterverarbeitet und in die entsprechende Form gebracht.

Die erfindungsgemäße Kautschukmischung ist besonders für die Verwendung in Fahrzeugreifen, insbesondere Fahrzeugluftreifen geeignet. Hierbei ist die Anwendung in allen Reifenbauteilen prinzipiell denkbar, insbesondere in einem äußeren Bauteil, insbesondere und bevorzugt im Hornprofil, Laufstreifen und/oder der Seitenwand Laufstreifen. Im Falle eines Laufstreifens mit Cap/Base-Konstruktion wird die erfindungsgemäße Kautschukmischung bevorzugt wenigstens in der Cap verwendet.

Zur Verwendung in Fahrzeugreifen wird die Mischung als Fertigmischung vor der Vulkanisation in die entsprechende Form, bevorzugt eines äußeren Bauteils, gebracht und bei der Herstellung des Fahrzeugreifenrohlings wie bekannt aufgebracht.

Die Herstellung der erfindungsgemäßen Kautschukmischung zur Verwendung als sonstige Body-Mischung in Fahrzeugreifen erfolgt wie bereits beschrieben. Der Unterschied liegt in der Formgebung nach dem Extrusionsvorgang bzw. dem Kalandrieren der Mischung. Die so erhaltenen Formen der noch unvulkanisierten Kautschukmischung für eine oder mehrere unterschiedliche Body-Mischungen dienen dann dem Aufbau eines Reifenrohlings.

Als Body-Mischung werden hierbei die Kautschukmischungen für die inneren Bauteile eines Reifen bezeichnet, wie im Wesentlichen Squeegee, Innenseele (Innenschicht), Kernprofil, Gürtel, Schulter, Gürtelprofil, Karkasse, Wulstverstärker, Wulstprofil, Hornprofil und Bandage.

Der noch unvulkanisierte Reifenrohling wird anschließend vulkanisiert.

Zur Verwendung der erfindungsgemäßen Kautschukmischung in Riemen und Gurten, insbesondere in Fördergurten, wird die extrudierte noch unvulkanisierte Mischung in die entsprechende Form gebracht und dabei oder nachher häufig mit Festigkeitsträgern, z.B. synthetische Fasern oder Stahlcorde, versehen. Zumeist ergibt sich so ein mehrlagiger Aufbau, bestehend aus einer und/oder mehrerer Lagen Kautschukmischung, einer und/oder mehrerer Lagen gleicher und/oder verschiedener Festigkeitsträger und einer und/oder mehreren weiteren Lagen dergleichen und/oder einer anderen Kautschukmischung.

Ein weiterer Gegenstand der vorliegenden Erfindung ist ein Fahrzeugreifen, der die erfindungsgemäße Kautschukmischung enthaltend die erfindungsgemäße Verbindung in wenigstens einem Bauteil aufweist.

Der vulkanisierte Fahrzeugreifen weist wenigstens in einem Bauteil ein Vulkanisat wenigstens einer erfindungsgemäßen Kautschukmischung auf. Dem Fachmann ist bekannt, dass die meisten Substanzen, wie z. B. die enthaltenen Kautschuke entweder bereits nach dem Mischen oder erst nach der Vulkanisation in chemisch veränderter Form vorliegen oder vorliegen können.

Unter Fahrzeugreifen werden im Rahmen der vorliegenden Erfindung Fahrzeugluftreifen und Vollgummireifen, inklusive Reifen für Industrie- und Baustellenfahrzeuge, LKW-, PKW- sowie Zweiradreifen verstanden.

Bevorzugt weist der erfindungsgemäße Fahrzeugreifen die erfindungsgemäße Kautschukmischung in wenigstens einem äußeren Bauteil auf, wobei das äußere Bauteil bevorzugt ein Laufstreifen, eine Seitenwand und/oder ein Hornprofil ist.

Der erfindungsgemäße Fahrzeugreifen kann die erfindungsgemäße Kautschukmischung enthaltend die erfindungsgemäße Verbindung gemäß Formel I) somit auch in mehreren Bauteilen in ggf. angepasster Zusammensetzung aufweisen.

Ein weiterer Aspekt der vorliegenden Erfindung ist ein Verfahren zur Herstellung der Verbindung gemäß Formel I), welches wenigstens die folgenden Verfahrensschritte umfasst:
a1) Bereitstellung der Substanzen gemäß Formel A1) und A2): 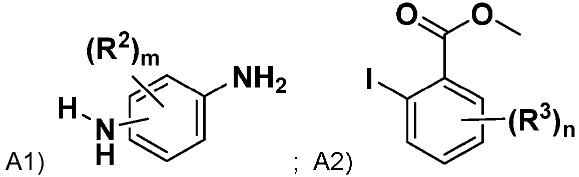
b1) Umsetzung der Substanzen aus Schritt a1) in Gegenwart einer Base, insbesondere einer anorganischen Base, die insbesondere ausgewählt ist aus der Gruppe bestehend aus Carbonaten, wie Kaliumcarbonat (K₂CO₃), Natriumcarbonat (Na₂CO₃), und Phosphaten, wie Kaliumphosphat (K₃PO₄), wobei Kaliumcarbonat besonders bevorzugt ist,
und eines Katalysators, insbesondere eines Katalysators auf Basis von Kupfer oder Palladium, wobei der Katalysator bevorzugt ausgewählt ist aus der Gruppe bestehend aus Kupfer (Cu), insbesondere Kupferpulver, Kupferhalogeniden, insbesondere Kupferiodid (Cul), Kupferbromid (CuBr), Kupferchlorid (CuCI), und Palladiumkomplexen, wobei Kupferiodid besonders bevorzugt ist, zu der Substanz gemäß Formel B1): 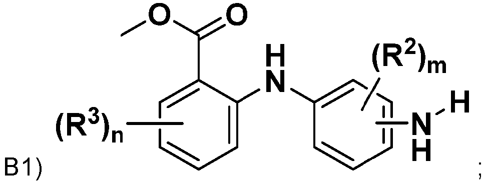
c1) Umsetzung der Verbindung gemäß Formel B1) mit Wasserstoff und einem Keton oder Aldehyd, bevorzugt Keton, zu der Verbindung gemäß Formel C1): 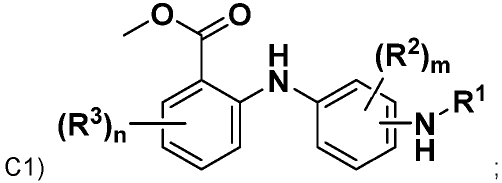
d1) Bereitstellung der Substanzen R⁴Li oder R⁴Mg und R⁵Li oder R⁵Mg, wobei R⁴Li und R⁵Li bevorzugt sind,
wobei R⁴ und R⁵ bevorzugt ausgewählt sind aus der Gruppe bestehend aus linearen, verzweigten und cyclischen aliphatischen C₁- bis C₁₂-Resten, wobei im Fall, dass R⁴ und R⁵ gleich sind, R⁴Li und R⁵Li bzw. R⁴Mg und R⁵Mg dieselbe Substanz sein können;
e1) Umsetzung der Substanz aus Schritt c1) mit der oder den Substanzen aus Schritt d1) zu der Verbindung gemäß Formel E1): 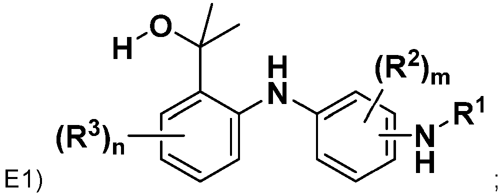
f1) Umsetzung der Substanz aus Schritt e1) mit einer Lewis-Säure, die bevorzugt ausgewählt ist aus der Gruppe bestehend aus Bor-Lewis-Säuren, wie insbesondere Bortrifluoriddiethyletherat, Methansulfonsäure, Polyphosphorsäure, Schwefelsäure, Salzsäure, Phosphonsäure, Trifluoressigsäure, einem Gemisch aus 1,2-bis(Ethenyl)benzol und 2-Ethenylbenzolsulfonsäure, welches insbesondere unter dem Handelsnamen Amberlyst^{™} 15 erhältlich ist, Bromwasserstoff (HBr), und para-Toluolsulfonsäure, wobei Bortrifluoriddiethyletherat besonders bevorzugt ist,
zu der Verbindung gemäß Formel I): 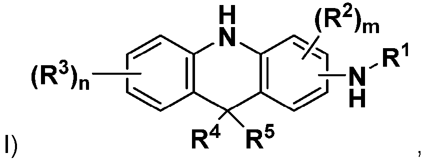
   wobei R¹ ausgewählt ist aus der Gruppe bestehend aus Benzyl- und linearen, verzweigten und cyclischen aliphatischen C₃- bis C₁₂-Resten; und
   wobei R³ ausgewählt ist aus der Gruppe bestehend aus linearen, verzweigten und cyclischen aliphatischen C₁- bis C₁₂-Resten, sowie Aryl-Resten, Cyano-Resten, Halogen-Resten, wobei Fluor, Brom und Chlor bevorzugt sind, Ester-Resten, Keton-Resten, Ether-Resten und Thioether-Resten, und wobei n den Wert 0 oder 1 oder 2 oder 3 oder 4 annimmt, wobei die Reste R³ im Fall von n gleich 2 oder 3 oder 4 unabhängig voneinander gleich oder verschieden sind, und
   wobei R² ausgewählt ist aus der Gruppe bestehend aus linearen, verzweigten und cyclischen aliphatischen C₁- bis C₁₂-Resten, sowie Aryl-Resten, Cyano-Resten, Halogen-Resten, wobei Fluor, Brom und Chlor bevorzugt sind, Ester-Resten, Keton-Resten, Ether-Resten und Thioether-Resten; und
   wobei m den Wert 0 oder 1 oder 2 oder 3 annimmt, wobei die Reste R² im Fall von m gleich 2 oder 3 unabhängig voneinander gleich oder verschieden sind, und wobei die Reste R⁴ und R⁵ unabhängig voneinander gleich oder verschieden sind und jeweils ausgewählt sind aus der Gruppe bestehend aus linearen, verzweigten und cyclischen aliphatischen C₁- bis C₁₂-Resten, sowie Aryl-Resten, Cyano-Resten, Halogen-Resten, wobei Fluor, Brom und Chlor bevorzugt sind, Ester-Resten, Keton-Resten, Ether-Resten und Thioether-Resten, wobei die Reste R⁴ und R⁵ unabhängig voneinander gleich oder verschieden sind und jeweils bevorzugt ausgewählt sind aus der Gruppe bestehend aus linearen, verzweigten und cyclischen aliphatischen C₁- bis C₁₂-Resten, wobei die linearen, verzweigten und cyclischen aliphatischen C₁- bis C₁₂-Reste sowie Aryl-Reste Substituenten tragen können.

Bevorzugt wird zudem bei der Umsetzung mit Wasserstoff ein geeigneter Katalysator, im Rahmen der vorliegenden Erfindung als "Hydrierungskatalysator" bezeichnet, verwendet.

Bevorzugt ist der Hydrierungskatalysator ein Edelmetallkatalysator, wie insbesondere Palladium (Pd) oder Platin (Pt). Bevorzugt wird das Edelmetall auf Kohle (C) eingesetzt, wie Palladium auf Kohle (Pd/C).

Ferner können auch andere bekannte Katalysatoren, wie Raney-Nickel oder Kupferchromit verwendet werden.

Besonders bevorzugt erfolgt damit die Umsetzung in Schritt c1) mit Wasserstoff. unter Verwendung eines Hydrierungskatalysators.

Bevorzugt erfolgt die Umsetzung in Schritt c1) bei einer Temperatur von 40 bis 100 °C, insbesondere zum Beispiel 60 °C.

Bevorzugt wird Wasserstoff mit einem Druck von 10 bis 30 bar, insbesondere zum Beispiel 20 bar, aufgedrückt und bevorzugt anschließend für 1 bis 20 Stunden, bevorzugt 3 bis 13 Stunden, besonders bevorzugt 5 bis 13 Stunden, insbesondere zum Beispiel 10 Stunden, gerührt.

Bevorzugt erfolgt die Umsetzung mit Wasserstoff in Schritt c1) in einem für den bevorzugt vergleichsweise hohen Druck geeigneten Behälter, wie insbesondere in einem Autoklav oder in einem anderem Druckreaktor.

Besonders bevorzugt erfolgt die Umsetzung in Schritt c1) mit Wasserstoff unter Verwendung eines Hydrierungskatalysators und bei einer Temperatur von 40 bis 100 °C und wobei Wasserstoff mit einem Druck von 10 bis 30 bar aufgedrückt wird und die Reaktion in einem Autoklav oder in einem anderen Druckreaktor stattfindet.

Bei dem Keton in Schritt c1) handelt es sich um das Keton-Derivat des späteren Restes R¹; Bei einem Aldehyd entsprechend um das Aldehyd-Derivat.

Der Einfachheit halber wird verkürzt die Formel R¹=O für das Aldehyd oder Keton verwendet, da der Rest R¹ der Teil ist, der nach der Reaktion mit dem Aldehyd oder Keton am Stickstoffatom verbleibt.

Bevorzugt wird hierbei das Keton Methylisobutylketon verwendet.

Das Lösungsmittel in Schritt c1) kann entweder das Keton oder Aldehyd sein, wenn dieses in flüssiger Form vorliegt, oder ein inertes Lösungsmittel, wie Toluol oder Xylol sein, insbesondere wenn das Keton oder Aldehyd in fester Form vorliegt. Im letzteren Fall wird das Keton oder Aldehyd nur in stöchiometrischen Mengen als Reaktant eingesetzt.

Bevorzugt wird ein Keton oder Aldehyd, besonders bevorzugt Keton, in flüssiger Form als Lösungsmittel verwendet. Hierdurch kann auf eine zusätzliche Substanz, wie Toluol oder Xylol, verzichtet werden.

Bevorzugt erfolgt im Anschluss an Schritt c1) eine Aufreinigung, wie beispielsweise säulenchromatographisch, beispielsweise an Kieselgel oder durch Umkristallisation aus Cyclohexan oder höherkettigen Aliphaten.

Ein alternatives Verfahren zur Herstellung der erfindungsgemäßen Verbindung gemäß Formel I), wobei die Reste R⁴ und R⁵ gleich und jeweils Methylreste sind, weist wenigstens die folgenden Verfahrensschritte gemäß den Schemata X1) und X2) auf:

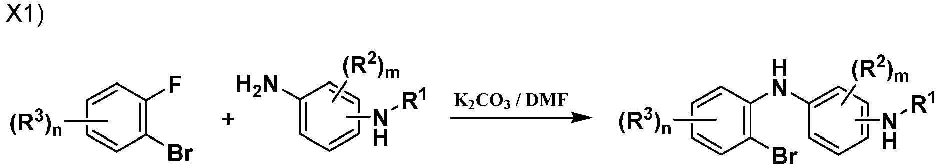

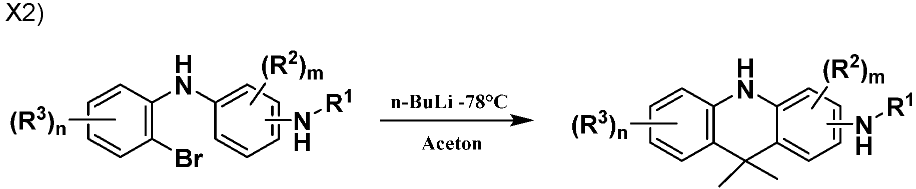

Wobei für die Reste R¹, R², R³ sowie die Indizes m und n sämtliche obige Ausführungen gelten.

Der Schritt X1) erfolgt insbesondere nach der Offenbarung der CN111269250 A1. Der Schritt X2) erfolgt insbesondere nach der Offenbarung der WO 2014017844 A1.

Ein weiteres alternatives Verfahren zur Herstellung der erfindungsgemäßen Verbindung gemäß Formel I) weist wenigstens die folgenden Verfahrensschritte gemäß den Schemata Y1), Y2) und Y3) auf: 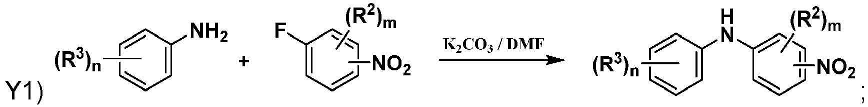 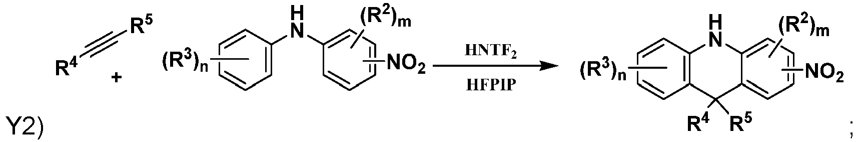 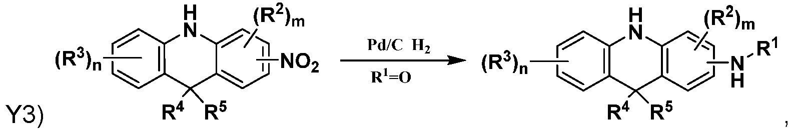 wobei HFPIP für Hexafluoro-2-propanol und HNTF₂ für Bis(trifluoromethan)sulfonamid steht, und wobei für die Reste R¹, R², R³, R⁴ und R⁵ sowie die Indizes m und n sämtliche obige Ausführungen zu Formel I) gelten.

Die Schritte Y1) und Y2) erfolgen dabei insbesondere nach S. Wang et al., Org. Lett. 2021, 23, 7, 2565-2570.

Bevorzugt erfolgt in Schritt Y3) die Umsetzung mit Wasserstoff und dem Keton R¹=O, bevorzugt Methyilisobutylketon (MIBK) unter den oben für Schritt c1) angegebenen Bedingungen.

Im Folgenden soll die Erfindung anhand von Ausführungsbeispielen näher erläutert werden.

Die Verbindung gemäß Formel II) als beispielhafte Ausführungsform der Verbindung gemäß Formel I) wurde auf folgende Weise gemäß einer ersten Syntheseroute hergestellt:

### Synthese von 2-(p-Phenylendiamin)-methylbenzoat:

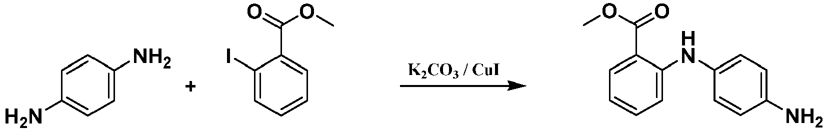

In 20 mL trockenem Dimethylsulfoxid (DMSO) wurden 1.6 g (14.5 mmol, 2.0 Äq) p-Phenylendiamin sowie 1.9 g 2-Iodmethylbenzoat (7.24 mmol, 1.0 Äq) vorgelegt. Nach der Zugabe von 1.00 g Kaliumcarbonat (K₂CO₃) (7.24 mmol, 1.0 Äq) und 0.14 g Kupferiodid (Cul) (0.72 mmol, 0.1 Äq) wurde über Nacht bei 80°C gerührt. Das Lösungsmittel wurde nach Beendigung der Reaktion destillativ entfernt und der Rückstand in einer Mischung aus Ethylacetat und 5%igem wässrigen Ammoniak aufgenommen. Die organische Phase wurde, bevor diese über Natriumsulfat getrocknet wurde, ein weiteres Mal mit 5%iger Ammoniaklösung, Wasser sowie gesättigter (ges.) Kochsalzlösung ausgeschüttelt. Die anorganischen Salze wurden mittels Filtration abgetrennt und das Lösungsmittel im Vakuum entfernt. Der Rückstand wurde durch Säulenchromatographie an Kieselgel (Dichlormethan (DCM) / Methanol (MeOH) 95:5) aufgereinigt: Es wurde ein oranges Öl erhalten; Ausbeute 1.6 g (91 % d. Th.).

¹H-NMR (*engl.* "nuclear magnetic resonance") (500 MHz, DMSO-d6) δ = 9.00 (s, 1H), 7.83 (dd, *J* = 8.6, 1.7 Hz, 1H), 7.29 (ddd, *J* = 8.6, 7.0, 1.7 Hz, 1H), 6.91 (d, *J* = 8.5 Hz, 2H), 6.79 (dd, *J* = 8.6, 1.1 Hz, 1H), 6.67 - 6.56 (m, 3H), 5.07 (s, 2H), 3.84 (s, 3H).

¹³C-NMR (126 MHz, DMSO-d6) δ = 168.7, 150.4, 146.9, 134.9, 128.4, 126.6, 116.0, 115.1, 113.4, 110.0, 52.2.

### Synthese von 2-(N¹-(4-Methylpentan-2-yl)-N⁴-p-phenylendiamin)-methylbenzoat:

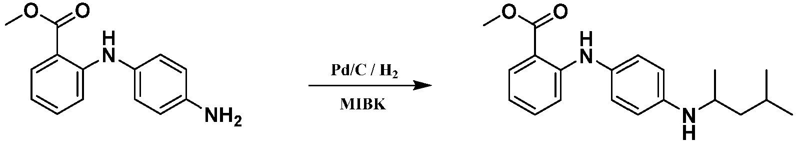

In einen Edelstahlautoklaven, der mit einem Tefloninliner bestückt wurde, wurden 6.80 g (28.1 mmol, 1 Äq) 2-(*p*-Phenylendiamin)-methylbenzoat, 1.18 g Palladium auf Kohle (Pd/C) (5%) (0.2 g auf 4.67 mmol Substrat) und 50.0 mL Methylisobutylketon (MIBK) eingewogen. Anschließend wurde 20 bar Wasserstoff (H₂) aufgedrückt und bei 60°C für 10 Stunden rühren gelassen. Nach Beendigung der Reaktion wurde der überschüssige Wasserstoff abgeblasen und die Suspension über Celite^{®} filtriert sowie mit Ethanol nachgewaschen. Das Filtrat wurde bis zur Trockne eingeengt und im Vakuum getrocknet. Der Rückstand wurde durch Säulenchromatographie an Kieselgel (Cyclohexan (CyHex) / Essigester (EE) 95:5) aufgereinigt. Oranges Öl; Ausbeute 8.20 g (89 % d. Th.).

¹H NMR (500 MHz, DMSO-*d*₆) δ = 9.01 (s, 1H), 7.83 (dd, *J* = 8.1, 1.7 Hz, 1H), 7.30 (ddd, *J* = 8.7, 7.0, 1.7 Hz, 1H), 6.95 (d, *J* = 8.7 Hz, 2H), 6.81 (dd, *J* = 8.6, 1.1 Hz, 1H), 6.66 - 6.56 (m, 3H), 5.32 (d, *J* = 8.6 Hz, 1H), 3.84 (s, 3H), 3.44 (dq, *J* = 8.6, 6.7 Hz, 1H), 1.74 (dt, *J* = 13.4, 6.7 Hz, 1H), 1.46 (dt, *J* = 14.0, 7.1 Hz, 1H), 1.27 - 1.16 (m, 1H), 1.09 (d, *J* = 6.2 Hz, 3H), 0.92 (d, *J* = 6.6 Hz, 3H), 0.88 (d, *J* = 6.6 Hz, 3H).

¹³C-NMR (126 MHz, DMSO-d6) δ = 168.7, 150.4, 146.6, 134.9, 131.6, 127.7, 126.8, 116.0, 113.4, 113.3, 109.9, 52.2, 46.4, 46.0, 25.00, 23.2, 23.1, 21.2.

ESI-MS (Elektrosprayionisation Massenspektrometrie) [M+H]⁺ = 327.

### Synthese von 2-(2-((4-((4-Methylpentan-2-yl)amino)phenyl)amino)phenyl)propan-2-ol:

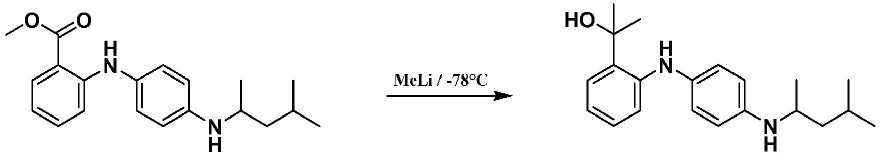

Es wurden 3.10 g 2-(*N*¹-(4-Methylpentan-2-yl)-*N*⁴-*p*-phenylendiamin)-methylbenzoat (9.5 mmol, 1 Äq) in 40 mL trockenem Diethylether gelöst und auf -78°C gekühlt. Nun wurden 17.81 mL (28.5 mmol, 3 Äq) Methyllithium (MeLi) langsam zugetropft, weitere 2 Stunden bei -78°C gerührt und über Nacht auf Raumtemperatur (RT) kommen gelassen. Die Reaktion wurde durch Zugabe von ges. Ammoniumchlorid-Lösung (NH₄Cl-Lsg.) abgebrochen. Die organische Phase wurde mit Wasser sowie ges. Kochsalzlösung ausgeschüttelt und über Natriumsulfat getrocknet. Die Salze wurden per Filtration und das Lösungsmittel im Vakuum entfernt. Aufgrund der hohen Reinheit wurde die Substanz ohne weitere Aufreinigung in der nächsten Stufe eingesetzt: Braunes bis schwärzliches Öl; Ausbeute 3.1 0 g (100 % d. Th.).

¹H-NMR (500 MHz, DMSO-*d*₆) δ = 8.06 (s, 1H), 7.13 (dd, *J* = 7.8, 1.6 Hz, 1H), 6.99 (ddd, *J* = 8.5, 7.2, 1.5 Hz, 1H), 6.90 - 6.81 (m, 3H), 6.61 (td, *J* = 7.4, 1.3 Hz, 1H), 6.58 - 6.51 (m, 2H), 5.66 (s, 1H), 4.99 (d, *J* = 8.7 Hz, 1H), 3.40 (dq, *J* = 7.1, 6.9 Hz, 1H), 1.74 (dh, *J* = 14.0, 6.9 Hz, 1H), 1.45 (dt, *J* = 13.9, 7.1 Hz, 1H), 1.21 (dt, *J* = 13.6, 6.8 Hz, 1H), 1.08 (d, *J* = 6.2 Hz, 3H), 0.92 (d, *J* = 6.7 Hz, 3H), 0.88 (d, *J* = 6.6 Hz, 3H).

¹³C-NMR (126 MHz, DMSO) δ = 145.8, 144.6, 133.0, 131.7, 127.7, 126.0, 123.6, 117.4, 114.3, 113.6, 72.9, 46.5, 46.1, 29.9, 25.0, 23.2, 23.1, 21.2.

ESI-MS [M+H]⁺ = 326.

### Synthese von 2-(N¹-(4-Methylpentan-2-yl)-N⁴-p-phenylendiamin)-benzoesäure:

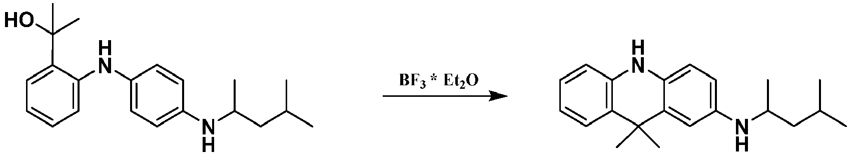

Es wurden 1.00 g 2-(2-((4-((4-Methylpentan-2-yl)amino)phenyl)amino)phenyl)propan-2-ol (3.06 mmol, 1 Äq) in 40 mL trockenem Ether gelöst und langsam 1.16 mL (9.18 mmol, 3Äq) Bortrifluoriddiethyletherat (BF₃*Et₂O) zugegeben. Über Nacht wurde die Reaktion bei RT gerührt. Durch Zugabe gesättigter NH₄Cl-Lösung wurde die Reaktion abgebrochen. Die organische Phase wurde mit gesättigter NaHCO₃-Lösung, Wasser sowie gesättigter Kochsalzlösung ausgeschüttelt und über Natriumsulfat getrocknet. Nachdem das Lösungsmittel im Vakuum entfernt wurde, wurde die Substanz mittels Chromatographie aufgereinigt (CyHex/EE 10:1). Dunkelbraunes, viskoses Öl; Ausbeute 0.05 g (5 % d. Th.).

¹H-NMR (500 MHz, DMSO-*d*₆) δ = 8.34 (s, 1H), 7.28 (dd, *J* = 7.8, 1.4 Hz, 1H), 6.99 (ddd, *J* = 8.3, 7.1, 1.4 Hz, 1H), 6.76 - 6.68 (m, 2H), 6.66 - 6.57 (m, 2H), 6.38 (dd, *J* = 8.4, 2.5 Hz, 1H), 4.56 (d, *J* = 5.7 Hz, 1H), 3.38 (br s, 1H, Überdeckt von H₂O-Peak), 1.74 (dp, *J* = 13.5, 6.7 Hz, 1H), 1.49 - 1.39 (m, 7H), 1.17 (dt, *J* = 13.6, 6.9 Hz, 1H), 1.07 (d, *J* = 6.1 Hz, 3H), 0.93 (d, *J* = 6.6 Hz, 3H), 0.88 (d, *J* = 6.6 Hz, 3H).

¹³C-NMR (126 MHz, DMSO) δ = 142.7, 140.4, 129.9, 129.3, 127.7, 126.7, 125.6, 118.8, 114.5, 113.4, 112.1, 110.6, 46.7, 46.6, 36.2, 31.1, 31.0, 26.8, 25.1, 23.5, 23.0, 21.3.

ESI-MS [M+H]⁺ = 309.

Alternativ kann die Verbindung gemäß Formel II) als beispielhafte Ausführungsform der Verbindung gemäß Formel I) auf folgende Weise hergestellt werden:

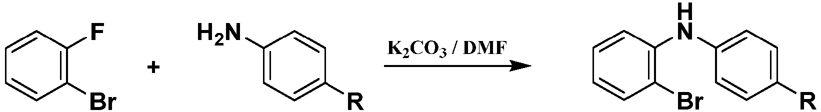

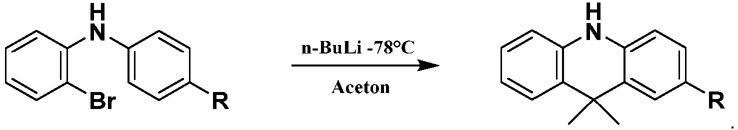

Die Verbindung gemäß Formel III) als beispielhafte Ausführungsform der Verbindung gemäß Formel I) kann auf folgende Weise hergestellt werden:

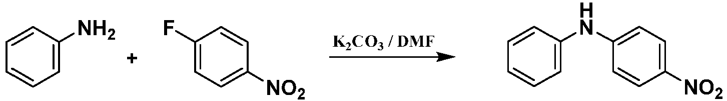

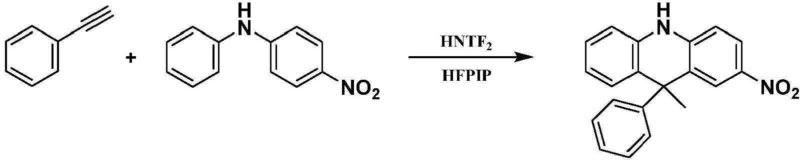

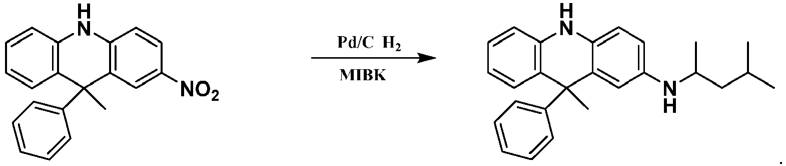

### Messung der Oxidations-Induktions-Zeit (OIT, engl. "oxidation induction time")

Die Verbindung gemäß Formel II) wurde durch Messung der Oxidations-Induktions-Zeit unter Laborbedingungen auf ihre potenzielle Schutzwirkung als Alterungsschutzmittel untersucht. Hierzu wurden die Verbindungen gemäß Formel II) sowie 6-PPD jeweils zusammen mit einem Polymer (flüssiges synthetisches Polyisopren (IR), LIR-50, Fa. Kuraray, Gewichtsmittel der Molekulargewichtsverteilung M_{w} = 54.000 g/mol, Glasübergangstemperatur T_{g} = -63°C) bei konstanter Temperatur (180°C) erhitzt bis Oxidation eintrat (Starttemperatur 35 °C, Aufheizen auf 170°C mit einer Heizrate von 20 K/min (Kelvin pro Minute), Aufheizen auf 180°C mit einer Heizrate von 1 K/min; Spülgas: Stickstoff (N₂) mit einem Volumenstrom von 50 mL/min). Die Probe wurde 5 Minuten bei 180°C isotherm unter N₂-Atmosphäre gehalten und anschließend wurde auf O₂-Atmosphäre (mit einem Volumenstrom von 50 mL/min) umgestellt.

Die Oxidation wurde über einen Peak mittels DSC (dynamische Differenzkalorimetrie; *engl.* "differential scanning calorimetry") ermittelt.

Gemessen wurde die Zeit in Minuten bis zur Oxidation.

Die Ergebnisse sind im Vergleich zu dem bekannten Alterungsschutzmittel 6PPD in Tabelle 1 zusammengefasst.

**Tabelle 1**

| **Substanz** | **Zeit [min] bei 180 °C** |
|---|---|
| 6PPD | 116 ± 10 |
| Formel II) | 111 ± 10 |

Unter Berücksichtigung der Messgenauigkeit von ± (Plusminus) 10 Minuten zeigt sich, dass die Verbindung gemäß Formel II) eine gegenüber 6PPD vergleichbare Schutzwirkung erzielt.

Auch bei 150 °C zeigt sich eine vergleichbare Schutzwirkung, wobei der Versuch jeweils für 6PPD bzw. die Verbindung gemäß Formel II) nach 900 Minuten abgebrochen wurde. Ansonsten erfolgte die Durchführung der Messung analog zur Messung bei 180 °C, wobei zunächst das Aufheizen auf 140°C mit einer Heizrate von 20 K/min (Kelvin pro Minute) erfolgte und anschließend das Aufheizen auf 150°C mit einer Heizrate von 1 K/min.

Damit ist die erfindungsgemäße Verbindung gemäß Formel II) als Vertreter der erfindungsgemäßen Verbindung gemäß Formel I) umwelt- und gesundheitsfreundlicher als 6-PPD bzw. weitere Vertreter der Substanzklasse, wie eingangs ausgeführt, und zudem ein besseres Alterungsschutzmittel.

Mit der Verbindung gemäß Formel I) kann somit eine vergleichbare Schutzwirkung bei den genannten Anwendungsmöglichkeiten erzielt werden.

Für die Anwendung in einer Kautschukmischung für Fahrzeugreifen wird die erfindungsgemäße Verbindung gemäß Formel I), beispielsweise gemäß Formel II), beispielsweise anstelle der im Stand der Technik bekannten Alterungsschutzmittel, wie 6PPD, 7PPD oder IPPD usw., auf dem Fachmann bekannte Weise in einer der Mischstufen bei der Herstellung der Kautschukmischung zugegeben.

Die Verbindung gemäß Formel II) wurde hiernach in verschiedenen Mengen in eine beispielhafte erfindungsgemäße Kautschukmischung, wie in Tabelle 2 gezeigt, eingemischt. Die sich ergebenden erfindungsgemäßen Beispiele sind mit E1 und E2 gekennzeichnet.

Als Vergleich dienen Kautschukmischungen enthaltend 6PPD anstelle der Verbindung gemäß Formel II) als Alterungsschutzmittel bei sonst gleicher Zusammensetzung, wobei zwischen V1 und E1 sowie V2 und E2 jeweils ein molgleicher Austausch stattfand. Die Mengen in Tabelle 2 sind in der Einheit phr angegeben. Ferner ist eine Referenz (Ref.) ohne Alterungsschutzmittel angegeben. Bei sämtlichen Mischungen beträgt die Summe der Mengen an Alterungsschutzmittel (6PPD oder Formel II) und Weichmacheröl MES 10 phr.

**Tabelle 2**

| **Bestandteil** | **V1** | **E1** | **V2** | **E2** | **Ref.** |
|---|---|---|---|---|---|
| NR | 100 | 100 | 100 | 100 | 100 |
| Ruß N 339 | 50 | 50 | 50 | 50 | 50 |
| MES | 8 | 7,71 | 5 | 4,26 | 10 |
| 6PPD | 2 | - | 5 | - | - |
| Verbindung gemäß Formel II) | - | 2,29 | - | 5,74 | - |
| ZnO | 3 | 3 | 3 | 3 | 3 |
| Stearinsäure | 2 | 2 | 2 | 2 | 2 |
| TBBS | 1,2 | 1,2 | 1,2 | 1,2 | 1,2 |
| Schwefel | 1,2 | 1,2 | 1,2 | 1,2 | 1,2 |

## Patentansprüche

1. Verbindung gemäß Formel I): 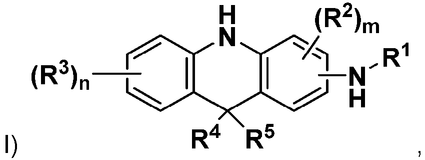
wobei R¹ ausgewählt ist aus der Gruppe bestehend aus Benzyl- und linearen, verzweigten und cyclischen aliphatischen C₃- bis C₁₂-Resten; und wobei R³ ausgewählt ist aus der Gruppe bestehend aus linearen, verzweigten und cyclischen aliphatischen C₁- bis C₁₂-Resten, sowie Aryl-Resten, Cyano-Resten, Halogen-Resten, wobei Fluor, Brom und Chlor bevorzugt sind, Ester-Resten, Keton-Resten, Ether-Resten und Thioether-Resten, und wobei n den Wert 0 oder 1 oder 2 oder 3 oder 4 annimmt, wobei die Reste R³ im Fall von n gleich 2 oder 3 oder 4 unabhängig voneinander gleich oder verschieden sind, und
wobei R² ausgewählt ist aus der Gruppe bestehend aus linearen, verzweigten und cyclischen aliphatischen C₁- bis C₁₂-Resten, sowie Aryl-Resten, Cyano-Resten, Halogen-Resten, wobei Fluor, Brom und Chlor bevorzugt sind, Ester-Resten, Keton-Resten, Ether-Resten und Thioether-Resten; und
wobei m den Wert 0 oder 1 oder 2 oder 3 annimmt, wobei die Reste R² im Fall von m gleich 2 oder 3 unabhängig voneinander gleich oder verschieden sind, und
wobei die Reste R⁴ und R⁵ unabhängig voneinander gleich oder verschieden sind und jeweils ausgewählt sind aus der Gruppe bestehend aus linearen, verzweigten und cyclischen aliphatischen C₁- bis C₁₂-Resten, sowie Aryl-Resten, Cyano-Resten, Halogen-Resten, wobei Fluor, Brom und Chlor bevorzugt sind, Ester-Resten, Keton-Resten, Ether-Resten und Thioether-Resten, wobei die linearen, verzweigten und cyclischen aliphatischen C₁- bis C₁₂-Reste sowie Aryl-Reste Substituenten tragen können.

2. Verbindung nach Anspruch 1, **dadurch gekennzeichnet, dass** n gleich 0 (null) ist.

3. Verbindung nach Anspruch 1 oder 2, **dadurch gekennzeichnet, dass** m gleich 0 (null) ist.

4. Verbindung nach einem der vorherigen Ansprüche, **dadurch gekennzeichnet, dass** R¹ über ein tertiäres Kohlenstoffatom an das Stickstoffatom (N) gebunden ist.

5. Verbindung nach einem der vorherigen Ansprüche, **dadurch gekennzeichnet, dass** R¹ ein verzweigter Alkyl-Rest mit 3 bis 12 Kohlenstoffatomen, bevorzugt 3 bis 8 Kohlenstoffatomen, ist.

6. Verbindung nach einem der vorherigen Ansprüche, **dadurch gekennzeichnet, dass** R¹ ausgewählt ist aus 1,3-Dimethylbutyl- und Cyclohexyl-Resten, wobei R¹ bevorzugt ein 1,3-Dimethylbutyl-Rest ist.

7. Verbindung nach einem der vorherigen Ansprüche, **dadurch gekennzeichnet, dass** die Reste R⁴ und R⁵ gleich sind und/oder jeweils ausgewählt sind aus der Gruppe bestehend aus nicht-substituierten, linearen, verzweigten und cyclischen aliphatischen C₁- bis C₁₂-Resten, und Aryl-Resten, wobei die Reste R⁴ und R⁵ dabei besonders bevorzugt ausgewählt sind aus der Gruppe bestehend aus Methyl-Resten, n-Butyl-Resten und Phenyl-Resten, wobei Methyl-Reste ganz besonders bevorzugt sind.

8. Verbindung nach einem der vorherigen Ansprüche, **dadurch gekennzeichnet, dass** sie die Struktur gemäß Formel II) aufweist:

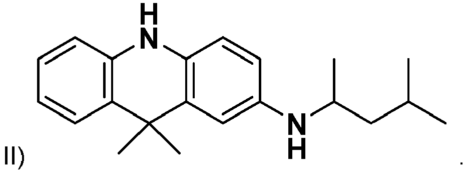

9. Kautschukmischung enthaltend die Verbindung nach einem der Ansprüche 1 bis 8, wobei die Kautschukmischung bevorzugt wenigstens einen Dienkautschuk enthält, der besonders bevorzugt ausgewählt ist aus der Gruppe bestehend aus natürlichem Polyisopren (NR), synthetischem Polyisopren (IR), Butadienkautschuk (BR), lösungspolymerisiertem Styrol-Butadien-Kautschuk (SSBR), emulsionspolymerisiertem Styrol-Butadien-Kautschuk (ESBR), Butylkautschuk (IIR) und Halobutylkautschuk.

10. Fahrzeugreifen, der die Kautschukmischung nach Anspruch 9 in wenigstens einem Bauteil, bevorzugt in wenigstens einem äußeren Bauteil, aufweist, wobei das äußere Bauteil bevorzugt ein Laufstreifen, eine Seitenwand und/oder ein Hornprofil ist.

11. Verwendung der Verbindung nach einem der Ansprüche 1 bis 8 als Alterungsschutzmittel und/oder Ozonschutzmittel insbesondere in Fahrzeugreifen und/oder anderen technischen Gummiartikeln, wie insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon, und/oder Ölen und/oder Schmierstoffen.

12. Verwendung der Verbindung nach einem der Ansprüche 1 bis 8 zur Herstellung eines Gummiartikels, insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon.

13. Verwendung der Verbindung nach einem der Ansprüche 1 bis 8 in Ölen, Schmierstoffen, wie insbesondere Treib- oder Betriebsstoffen für Motoren.

14. Verwendung der Verbindung nach einem der Ansprüche 1 bis 8 als Farbstoff in Fasern und/oder Polymeren und/oder Papier und/oder in (Streich-) Farben und Lacken.

15. Verfahren zur Herstellung der Verbindung gemäß Formel I), welches die folgenden Verfahrensschritte umfasst:
a1) Bereitstellung der Substanzen gemäß Formel A1) und A2): 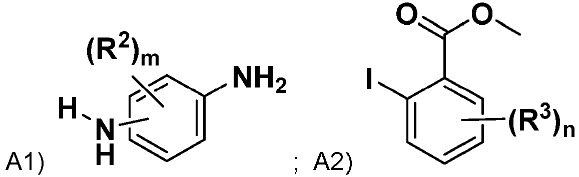
b1) Umsetzung der Substanzen aus Schritt a1) in Gegenwart einer Base, besonders bevorzugt Kaliumcarbonat, und eines Katalysators, besonders bevorzugt Kupferiodid, zu der Substanz gemäß Formel B1): 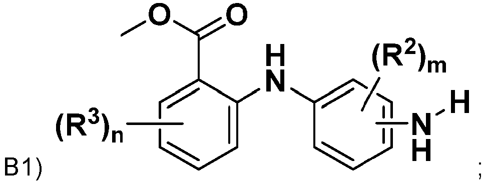
c1) Umsetzung der Verbindung gemäß Formel B1) mit Wasserstoff und einem Keton oder Aldehyd, bevorzugt Keton, zu der Verbindung gemäß Formel C1): 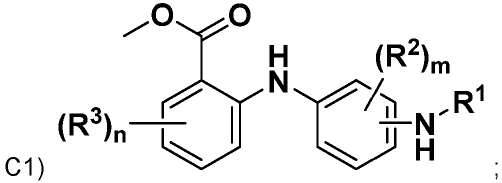
d1) Bereitstellung der Substanzen R⁴Li oder R⁴Mg und R⁵Li oder R⁵Mg, wobei R⁴Li und R⁵Li bevorzugt sind,
wobei R⁴ und R⁵ bevorzugt ausgewählt sind aus der Gruppe bestehend aus linearen, verzweigten und cyclischen aliphatischen C₁- bis C₁₂-Resten, wobei im Fall, dass R⁴ und R⁵ gleich sind, R⁴Li und R⁵Li bzw. R⁴Mg und R⁵Mg dieselbe Substanz sein können;
e1) Umsetzung der Substanz aus Schritt c1) mit der oder den Substanzen aus Schritt d1) zu der Verbindung gemäß Formel E1): 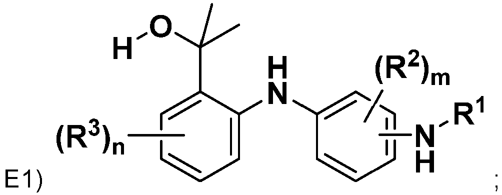
f1) Umsetzung der Substanz aus Schritt e1) mit einer Lewis-Säure, die bevorzugt ausgewählt ist aus der Gruppe bestehend aus Bor-Lewis-Säuren, wie insbesondere Bortrifluoriddiethyletherat, Methansulfonsäure, Polyphosphorsäure, Schwefelsäure, Salzsäure, Phosphonsäure, Trifluoressigsäure, einem Gemisch aus 1,2-bis(Ethenyl)benzol und 2-Ethenylbenzolsulfonsäure, welches insbesondere unter dem Handelsnamen Amberlyst^{™} 15 erhältlich ist, Bromwasserstoff (HBr), und para-Toluolsulfonsäure, wobei Bortrifluoriddiethyletherat besonders bevorzugt ist,
zu der Verbindung gemäß Formel I): 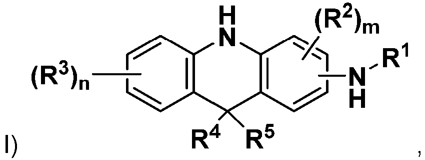
wobei R¹ ausgewählt ist aus der Gruppe bestehend aus Benzyl- und linearen, verzweigten und cyclischen aliphatischen C₃- bis C₁₂-Resten; und wobei R³ ausgewählt ist aus der Gruppe bestehend aus linearen, verzweigten und cyclischen aliphatischen C₁- bis C₁₂-Resten, sowie Aryl-Resten, Cyano-Resten, Halogen-Resten, wobei Fluor, Brom und Chlor bevorzugt sind, Ester-Resten, Keton-Resten, Ether-Resten und Thioether-Resten, und wobei n den Wert 0 oder 1 oder 2 oder 3 oder 4 annimmt, wobei die Reste R³ im Fall von n gleich 2 oder 3 oder 4 unabhängig voneinander gleich oder verschieden sind, und
wobei R² ausgewählt ist aus der Gruppe bestehend aus linearen, verzweigten und cyclischen aliphatischen C₁- bis C₁₂-Resten, sowie Aryl-Resten, Cyano-Resten, Halogen-Resten, wobei Fluor, Brom und Chlor bevorzugt sind, Ester-Resten, Keton-Resten, Ether-Resten und Thioether-Resten; und
wobei m den Wert 0 oder 1 oder 2 oder 3 annimmt, wobei die Reste R² im Fall von m gleich 2 oder 3 unabhängig voneinander gleich oder verschieden sind, und wobei die Reste R⁴ und R⁵ unabhängig voneinander gleich oder verschieden sind und jeweils ausgewählt sind aus der Gruppe bestehend aus linearen, verzweigten und cyclischen aliphatischen C₁- bis C₁₂-Resten, sowie Aryl-Resten, Cyano-Resten, Halogen-Resten, wobei Fluor, Brom und Chlor bevorzugt sind, Ester-Resten, Keton-Resten, Ether-Resten und Thioether-Resten, wobei die Reste R⁴ und R⁵ unabhängig voneinander gleich oder verschieden sind und jeweils bevorzugt ausgewählt sind aus der Gruppe bestehend aus linearen, verzweigten und cyclischen aliphatischen C₁- bis C₁₂-Resten, bevorzugt bestehend aus linearen C₁- bis C₁₂-Resten, besonders bevorzugt bestehend aus linearen C₁- bis C₆-Resten, wobei Methyl-Reste ganz besonders bevorzugt sind, wobei die linearen,
verzweigten und cyclischen aliphatischen C₁- bis C₁₂-Reste sowie Aryl-Reste Substituenten tragen können.

## Claims

1. Compound of formula I): 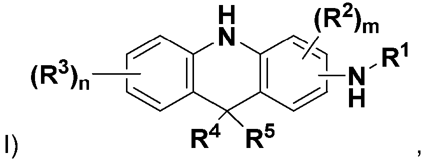
where R¹ is selected from the group consisting of benzyl and linear, branched and cyclic aliphatic C₃ to C₁₂ radicals; and
where R³ is selected from the group consisting of linear, branched and cyclic aliphatic C₁ to C₁₂ radicals, and aryl radicals, cyano radicals, halogen radicals, preferably fluorine, bromine and chlorine, ester radicals, ketone radicals, ether radicals and thioether radicals, and where n assumes the value of 0 or 1 or 2 or 3 or 4, where the R³ radicals, in the case that n = 2 or 3 or 4, are independently the same or different, and
where R² is selected from the group consisting of linear, branched and cyclic aliphatic C₁ to C₁₂ radicals, and aryl radicals, cyano radicals, halogen radicals, preferably fluorine, bromine and chlorine, ester radicals, ketone radicals, ether radicals and thioether radicals; and
where m assumes the value of 0 or 1 or 2 or 3, where the R² radicals, in the case that m = 2 or 3, are independently the same or different, and
where the R⁴ and R⁵ radicals are independently the same or different and are each selected from the group consisting of linear, branched and cyclic aliphatic C₁ to C₁₂ radicals, and aryl radicals, cyano radicals, halogen radicals, preferably fluorine, bromine and chlorine, ester radicals, ketone radicals, ether radicals and thioether radicals, where the linear, branched and cyclic aliphatic C₁ to C₁₂ radicals and aryl radicals
may bear substituents.

2. Compound according to Claim 1, **characterized in that** n is 0 (zero).

3. Compound according to Claim 1 or 2, **characterized in that** m is 0 (zero).

4. Compound according to any of the preceding claims, **characterized in that** R¹ is bonded to the nitrogen atom (N) via a tertiary carbon atom.

5. Compound according to any of the preceding claims, **characterized in that** R¹ is a branched alkyl radical having 3 to 12 carbon atoms, preferably 3 to 8 carbon atoms.

6. Compound according to any of the preceding claims, **characterized in that** R¹ is selected from 1,3-dimethylbutyl and cyclohexyl radicals, where R¹ is preferably a 1,3-dimethylbutyl radical.

7. Compound according to any of the preceding claims, **characterized in that** the R⁴ and R⁵ radicals are the same and/or are each selected from the group consisting of unsubstituted, linear, branched and cyclic aliphatic C₁ to C₁₂ radicals, and aryl radicals, where the R⁴ and R⁵ radicals are more preferably selected from the group consisting of methyl radicals, n-butyl radicals and phenyl radicals, most preferably methyl radicals.

8. Compound according to any of the preceding claims, **characterized in that** it has the structure of formula II):

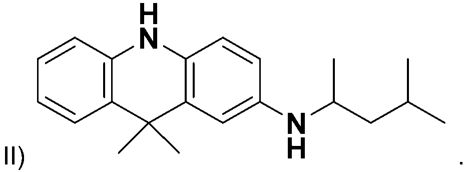

9. Rubber mixture comprising the compound according to any of Claims 1 to 8, wherein the rubber mixture preferably contains at least one diene rubber which is particularly preferably selected from the group consisting of natural polyisoprene (NR), synthetic polyisoprene (IR), butadiene rubber (BR), solution-polymerized styrene-butadiene rubber (SSBR), emulsion-polymerized styrene-butadiene rubber (ESBR), butyl rubber (IIR) and halobutyl rubber.

10. Vehicle tyre including the rubber mixture according to Claim 9 in at least one component, preferably in at least one external component, where the external component is preferably a tread, a sidewall and/or a flange profile.

11. Use of the compound according to any of Claims 1 to 8 as aging stabilizer and/or antiozonant especially in vehicle tyres and/or other industrial rubber articles, such as, in particular, an air spring, a bellows, conveyor belt, strap, drive belt, hose, rubber band, profile, a seal, a membrane, tactile sensors for medical applications or robotics applications, or a shoe sole or parts thereof, and/or oils and/or lubricants.

12. Use of the compound according to any of Claims 1 to 8 for production of a rubber article, in particular an air spring, a bellows, conveyor belt, strap, drive belt, hose, rubber band, profile, a seal, a membrane, tactile sensors for medical applications or robotics applications, or a shoe sole or parts thereof.

13. Use of the compound according to any of Claims 1 to 8 in oils, lubricants, such as, in particular, fuels or operating media for engines.

14. Use of the compound according to any of Claims 1 to 8 as a dye in fibres and/or polymers and/or paper and/or in (decorating) paints and coatings.

15. Process for preparing the compound of formula I) which comprises the following process steps:
a1) providing the substances of formula A1) and A2): 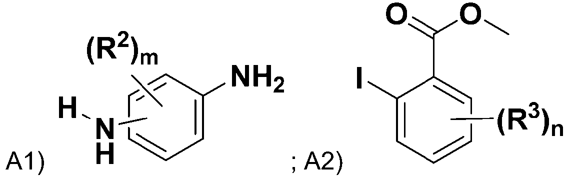
b1) converting the substances from step a1) in the presence of a base, more preferably potassium carbonate, and of a catalyst, more preferably copper iodide, to the substance of formula B1): 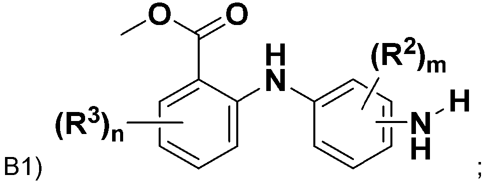
c1) reacting the compound of formula B1) with hydrogen and a ketone or aldehyde, preferably ketone, to give the compound of formula C1): 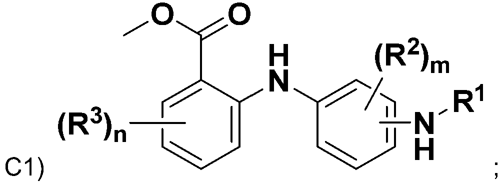
d1) providing the substances R⁴Li or R⁴Mg and R⁵Li or R⁵Mg, preferably R⁴Li and R⁵Li,
where R⁴ and R⁵ are preferably selected from the group consisting of linear, branched and cyclic aliphatic C₁ to C₁₂ radicals, where, in the case that R⁴ and R⁵ are the same, R⁴Li and R⁵Li or R⁴Mg and R⁵Mg may be the same substance; e1) reacting the substance from step c1) with the substance(s) from step d1) to give the compound of formula E1): 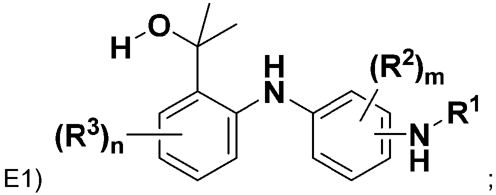
f1) reacting the substance from step e1) with a Lewis acid, preferably selected from the group consisting of boron Lewis acids, such as, in particular, boron trifluoride diethyletherate, methanesulfonic acid, polyphosphoric acid, sulfuric acid, hydrochloric acid, phosphonic acid, trifluoroacetic acid, a mixture of 1,2-bis(ethenyl)benzene and 2-ethenylbenzenesulfonic acid, especially obtainable under the Amberlyst^{™} 15 trade name, hydrogen bromide (HBr), and paratoluenesulfonic acid, more preferably boron trifluoride diethyletherate, to give the compound of formula I): 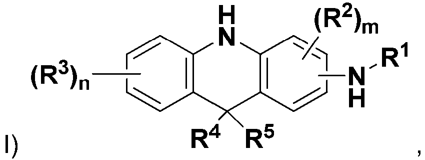
where R¹ is selected from the group consisting of benzyl and linear, branched and cyclic aliphatic C₃ to C₁₂ radicals; and
where R³ is selected from the group consisting of linear, branched and cyclic aliphatic C₁ to C₁₂ radicals, and aryl radicals, cyano radicals, halogen radicals, preferably fluorine, bromine and chlorine, ester radicals, ketone radicals, ether radicals and thioether radicals, and where n assumes the value of 0 or 1 or 2 or 3 or 4, where the R³ radicals, in the case that n = 2 or 3 or 4, are independently the same or different, and
where R² is selected from the group consisting of linear, branched and cyclic aliphatic C₁ to C₁₂ radicals, and aryl radicals, cyano radicals, halogen radicals, preferably fluorine, bromine and chlorine, ester radicals, ketone radicals, ether radicals and thioether radicals; and
where m assumes the value of 0 or 1 or 2 or 3, where the R² radicals, in the case that m = 2 or 3, are independently the same or different, and where the R⁴ and R⁵ radicals are independently the same or different and are each selected from the group consisting of linear, branched and cyclic aliphatic C₁ to C₁₂ radicals, and aryl radicals, cyano radicals, halogen radicals, preferably fluorine, bromine and chlorine, ester radicals, ketone radicals, ether radicals and thioether radicals, where the R⁴ and R⁵ radicals are independently the same or different and are each preferably selected from the group consisting of linear, branched and cyclic aliphatic C₁ to C₁₂ radicals, preferably consisting of linear C₁ to C₁₂ radicals, more preferably consisting of linear C₁ to C₆ radicals, most preferably methyl radicals, where the linear, branched and cyclic aliphatic C₁ to C₁₂ radicals and aryl radicals may bear substituents.

## Revendications

1. Composé selon la formule (I) : 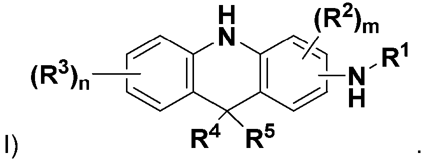
dans laquelle R¹ est choisi dans le groupe constitué par les radicaux benzyle et aliphatiques en C₃ à C₁₂ linéaires, ramifiés et cycliques ; et
dans laquelle R³ est choisi dans le groupe constitué par les radicaux aliphatiques en C₁ à C₁₂ linéaires, ramifiés et cycliques, ainsi que les radicaux aryle, les radicaux cyano, les radicaux halogène, le fluor, le brome et le chlore étant préférés, les radicaux ester, les radicaux cétone, les radicaux éther et les radicaux thioéther, et dans laquelle n prend la valeur 0 ou 1 ou 2 ou 3 ou 4, les radicaux R³ étant identiques ou différents indépendamment les uns des autres dans le cas où n est égal à 2 ou 3 ou 4, et
dans laquelle R² est choisi dans le groupe constitué par les radicaux aliphatiques en C₁ à C₁₂ linéaires, ramifiés et cycliques, ainsi que les radicaux aryle, les radicaux cyano, les radicaux halogène, le fluor, le brome et le chlore étant préférés, les radicaux ester, les radicaux cétone, les radicaux éther et les radicaux thioéther ; et
dans laquelle m prend la valeur 0 ou 1 ou 2 ou 3, les radicaux R² étant identiques ou différents indépendamment les uns des autres dans le cas où m est égal à 2 ou 3, et
dans laquelle les radicaux R⁴ et R⁵ sont, indépendamment les uns des autres, identiques ou différents et sont choisis chacun dans le groupe constitué par les radicaux aliphatiques en C₁ à C₁₂ linéaires, ramifiés et cycliques, ainsi que les radicaux aryle, les radicaux cyano, les radicaux halogène, le fluor, le brome et le chlore étant préférés, les radicaux ester, les radicaux cétone, les radicaux éther et les radicaux thioéther, les radicaux aliphatiques en C₁ à C₁₂ linéaires, ramifiés et cycliques ainsi que les radicaux aryle pouvant porter des substituants.

2. Composé selon la revendication 1, **caractérisé en ce que** n est égal à 0 (zéro).

3. Composé selon la revendication 1 ou 2, **caractérisé en ce que** m est égal à 0 (zéro).

4. Composé selon l'une quelconque des revendications précédentes, **caractérisé en ce que** R¹ est lié à l'atome d'azote (N) par un atome de carbone tertiaire.

5. Composé selon l'une quelconque des revendications précédentes, **caractérisé en ce que** R¹ est un radical alkyle ramifié ayant de 3 à 12 atomes de carbone, de préférence 3 à 8 atomes de carbone.

6. Composé selon l'une quelconque des revendications précédentes, **caractérisé en ce que** R¹ est choisi parmi les radicaux 1,3-diméthylbutyle et cyclohexyle, R¹ étant de préférence un radical 1,3-diméthylbutyle.

7. Composé selon l'une quelconque des revendications précédentes, **caractérisé en ce que** les radicaux R⁴ et R⁵ sont identiques et/ou sont choisis chacun dans le groupe constitué par les radicaux aliphatiques en C₁ à C₁₂ linéaires, ramifiés et cycliques non substitués, et les radicaux aryle, les radicaux R⁴ et R⁵ étant choisis de façon particulièrement préférée dans le groupe constitué par les radicaux méthyle, les radicaux n-butyle et es radicaux phényle, les radicaux méthyle étant tout particulièrement préférés.

8. Composé selon l'une quelconque des revendications précédentes, **caractérisé en ce qu'**il présente la structure selon la formule II) :

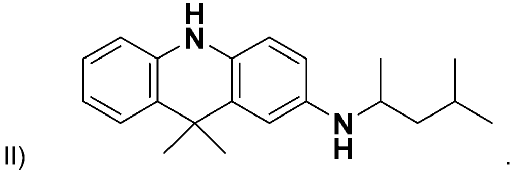

9. Composition de caoutchouc contenant le composé selon l'une quelconque des revendications 1 à 8, la composition de caoutchouc contenant de préférence au moins un caoutchouc diénique, qui est de façon particulièrement préférée choisi dans le groupe constitué par le polyisoprène naturel (NR), le polyisoprène synthétique (IR), le caoutchouc butadiène (BR), le caoutchouc styrène-butadiène polymérisé en solution (SSBR), le caoutchouc styrène-butadiène polymérisé en émulsion (ESBR), le caoutchouc butyle (IIR) et le caoutchouc halogénobutyle.

10. Pneumatique pour véhicule, qui comporte la composition de caoutchouc selon la revendication 9 dans au moins un composant, de préférence dans au moins un composant externe, le composant externe étant de préférence une bande de roulement, un flanc latéral et/ou un profilé de talon.

11. Utilisation du composé selon la formule I) selon l'une quelconque des revendications 1 à 8 en tant qu'agent de protection contre le vieillissement et/ou agent de protection contre l'ozone, en particulier dans des pneumatiques de véhicule et/ou d'autres articles techniques en caoutchouc, tels qu'en particulier un ressort pneumatique, un soufflet, une bande transporteuse, une sangle, une courroie, un tuyau, une bande en caoutchouc, un profilé, un joint d'étanchéité, une membrane, des capteurs tactiles pour applications médicales ou applications robotiques, ou une semelle de chaussure ou de parties de celle-ci, et/ou des huiles et/ou des lubrifiants.

12. Utilisation du composé selon l'une quelconque des revendications 1 à 8 pour la fabrication d'un article en caoutchouc, en particulier d'un ressort pneumatique, d'un soufflet, d'une bande transporteuse, d'une sangle, d'une courroie, d'un tuyau, d'une bande en caoutchouc, d'un profilé, d'un joint d'étanchéité, d'une membrane, de capteurs tactiles pour applications médicales ou applications robotiques, ou d'une semelle de chaussure ou de parties de celle-ci.

13. Utilisation du composé selon l'une quelconque des revendications 1 à 8 dans des huiles, des lubrifiants, tels qu'en particulier des carburants ou fluides opérationnels pour moteurs.

14. Utilisation du composé selon l'une quelconque des revendications 1 à 8 en tant que colorant dans des fibres et/ou des polymères et/ou du papier et/ou des peintures et vernis (à appliquer par étalement).

15. Procédé pour la préparation du composé selon la formule I), qui comprend les étapes de procédé suivantes :
a1) mise à disposition des substances selon lest formules A1) et A2) : 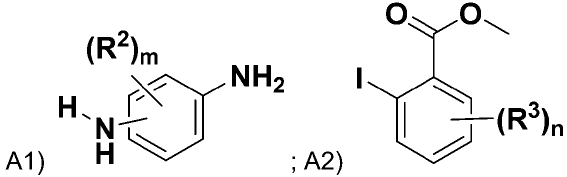
b1) mise en réaction des substances provenant de l'étape a1) en présence d'une base, de façon particulièrement préférée de carbonate de potassium, et d'un catalyseur, de façon particulièrement préférée de l'iodure de cuivre, pour obtenir la substance selon la formule B1) : 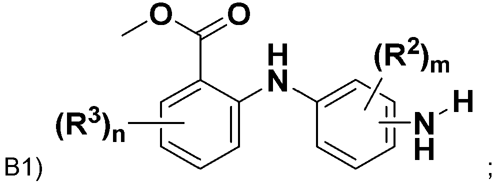
c1) mise en réaction du composé selon la formule B1) avec de l'hydrogène et une cétone ou un aldéhyde, de préférence une cétone, pour obtenir le composé selon la formule C1) : 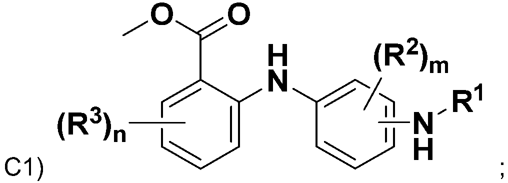
d1) mise à disposition des substances R⁴Li ou R⁴Mg et R⁵Li ou R⁵Mg, R⁴Li et R⁵Li étant préférés,
R⁴ et R⁵ étant choisis de préférence dans le groupe constitué par les radicaux aliphatiques en C₁ à C₁₂ linéaires, ramifiés et cycliques, dans le cas où R⁴ et R⁵ sont identiques, R⁴Li et R⁵Li ou, respectivement, R⁴Mg et R⁵Mg pouvant être la même substance ;
e1) mise en réaction de la substance provenant de l'étape c1 avec la ou les substances provenant de l'étape d1) pour aboutir au composé selon la formule E1) : 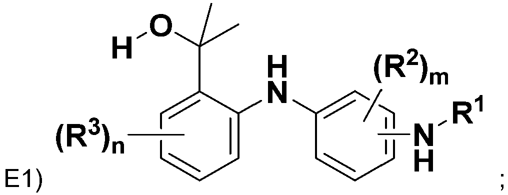
f1) mise en réaction de la substance provenant de l'étape e1) avec un acide de Lewis qui est choisi de préférence dans le groupe constitué par les acides de Lewis boriques, comme en particulier le diéthylétherate de trifluorure de bore, l'acide méthanesulfonique, l'acide polyphosphorique, l'acide sulfurique, l'acide chlorhydrique, l'acide phosphonique, l'acide trifluoroacétique, un mélange de 1,2-bis(éthényl)benzène et d'acide 2-éthénylbenzènesulfonique, qui peut être obtenu en particulier sous le nom commercial Amberlyst^{™} 15, le bromure d'hydrogène (HBr), et l'acide para-toluènesulfonique, le diéthylétherate de trifluorure de bore étant particulièrement préféré,
pour aboutir au composé selon la formule I) : 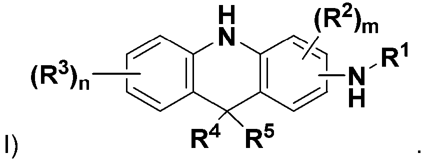
dans laquelle R¹ est choisi dans le groupe constitué par les radicaux benzyle et aliphatiques en C₃ à C₁₂ linéaires, ramifiés et cycliques ; et
dans laquelle R³ est choisi dans le groupe constitué par les radicaux aliphatiques en C₁ à C₁₂ linéaires, ramifiés et cycliques, ainsi que les radicaux aryle, les radicaux cyano, les radicaux halogène, le fluor, le brome et le chlore étant préférés, les radicaux ester, les radicaux cétone, les radicaux éther et les radicaux thioéther, et dans laquelle n prend la valeur 0 ou 1 ou 2 ou 3 ou 4, les radicaux R³ étant identiques ou différents indépendamment les uns des autres dans le cas où n est égal à 2 ou 3 ou 4, et
dans laquelle R² est choisi dans le groupe constitué par les radicaux aliphatiques en C₁ à C₁₂ linéaires, ramifiés et cycliques, ainsi que les radicaux aryle, les radicaux cyano, les radicaux halogène, le fluor, le brome et le chlore étant préférés, les radicaux ester, les radicaux cétone, les radicaux éther et les radicaux thioéther ; et
dans laquelle m prend la valeur 0 ou 1 ou 2 ou 3, dans laquelle les radicaux R², dans le cas où m est égal à 2 ou 3, sont indépendamment les uns des autres identiques ou différents, et dans laquelle les radicaux R⁴ et R⁵ sont indépendamment les uns des autres identiques ou différents et sont choisis chacun dans le groupe constitué par les radicaux aliphatiques en C₁ à C₁₂ linéaires, ramifiés et cycliques, ainsi que les radicaux aryle, les radicaux cyano, les radicaux halogène, le fluor, le brome et le chlore étant préférés, les radicaux ester, les radicaux cétone, les radicaux éther et les radicaux thioéther, dans laquelle les radicaux R⁴ et R⁵ sont, indépendamment l'un de l'autre, identiques ou différents et sont choisis chacun de préférence dans le groupe constitué par les radicaux aliphatiques en C₁ à C₁₂ linéaires, ramifiés et cycliques, de préférence constitué par les radicaux linéaires en C₁ à C₁₂, de façon particulièrement préférée constitué par les radicaux linéaires en C₁ à C₆, les radicaux méthyle étant tout particulièrement préférés, les radicaux aliphatiques en C₁ à C₁₂ linéaires, ramifiés et cycliques ainsi que les radicaux aryle pouvant porter des substituants.
